# EUROPEAN PATENT SPECIFICATION

(11) **EP 3 641 774 B1**
(45) Date of publication and mention of the grant of the patent: **27.03.2024**
(21) Application number: 18820988.6
(22) Date of filing: 20.06.2018
(51) Int. Cl.: A61K 31/53, A61K 36/232, A61K 36/73, A61K 36/752, A61K 47/02, A61K 47/12, A61K 47/26, A61K 47/42, A61K 47/44, A61K 9/68, A61K 31/05, A61K 31/135, A61K 47/36, A61K 31/485, A61K 31/495, A61K 31/4402

(54) **PECTIN GUMMY COMPOSITION AND METHODS OF MAKING AND USING THEREOF**
PEKTINGUMMIZUSAMMENSETZUNGEN UND VERFAHREN ZUR HERSTELLUNG UND VERWENDUNG DAVON
COMPOSITION GÉLIFIÉE À BASE DE PECTINE ET LEURS PROCÉDÉS DE FABRICATION ET D'UTILISATION

(30) Priority: 20.06.2017 US 201762522586 P; 20.06.2017 US 201762522552 P
(43) Date of publication of application: 29.04.2020
(73) Proprietor: Seattle Gummy Company, Seattle, Washington 98104 (US)
(72) Inventor: WAN, Feng, Issaquah Washington 98029 (US); CARLSON, William Brenden, Shoreline Washington 98155 (US)
(74) Representative: Hobson, David James
(86) International application number: PCT/US2018/038498
(87) International publication number: WO 2018/237000

(56) References cited:
- EP-A2- 2 431 028
- WO-A1-2009/102575
- WO-A1-2016/037745
- WO-A1-2016/176398
- WO-A1-2018/027070
- CA-A1- 2 843 610
- CN-A- 1 919 908
- FR-A1- 2 843 301
- US-A- 4 065 614
- US-A- 4 065 614
- US-A- 5 932 245
- US-A1- 2006 205 682
- US-A1- 2007 259 957
- US-A1- 2007 275 129
- US-A1- 2010 210 732
- US-A1- 2010 226 904
- US-A1- 2012 289 611
- US-A1- 2013 005 740
- US-A1- 2013 295 264
- US-A1- 2015 351 422
- US-A1- 2016 151 279
- US-B1- 6 709 669
- GIULIA AURIEMMA ET AL: "Prilling for the development of multi-particulate colon drug delivery systems: Pectin vs. pectin-alginate beads", CARBOHYDRATE POLYMERS, vol. 92, no. 1, 1 January 2013 (2013-01-01), pages 367-373, XP055767300, GB ISSN: 0144-8617, DOI: 10.1016/j.carbpol.2012.09.056
- SINITSYA A ET AL: "Amidation of highly methoxylated citrus pectin with primary amines", CARBOHYDRATE POLYMERS, APPLIED SCIENCE PUBLISHERS, LTD. BARKING, GB, vol. 42, no. 4, 22 May 2017 (2017-05-22), pages 359-368, XP085006535, ISSN: 0144-8617, DOI: 10.1016/S0144-8617(99)00184-8
- OMRI: "Citric Acid and Salts. Handling/Processing", Technical Evaluation Report, 17 February 2015 (2015-02-17), XP055555006, Retrieved from the Internet: URL:https://www.ams.usda.gov/sites/default /files/media/Citric%20Acid%20 TR %202015.pdf [retrieved on 2018-08-09]

## Description

### TECHNICAL FIELD

The application relate generally to gummy composition or formula, methods of making, using, or administration gummy compositions, and kits comprising gummy compositions.

### BACKGROUND

Pharmaceuticals are available in a variety of dosage forms for treating the diseases. Dosages that are formulated to take orally including tablets, capsules, soft-gels, powders, chewable tablets, and liquid suspensions.

Tablets, capsules and soft-gels are difficult for individuals who have difficulties swallowing pills. This problem is magnified when the medications need to be taken 2-4 times per day to provide the desired therapeutic effect. Moreover, the need for a source of water or other liquid to assist with swallowing solid dosage forms can complicate administration.

Powders are often difficult to administrator and chewable tablets can be hard to chew especially for seniors and young children. In addition, powders and chewable tablets often have an unpleasant after-taste.

Liquid suspensions or solutions are sometimes used as an alternative to solid oral dosage forms. However, the dosing with liquid dosage forms is not precise, which can lead to the administration of too little or too much medications. In addition, liquid dosage forms are messy and often have a bitter taste, which could impact person compliance.

These issues relating to the traditional dosage form can lead to sub-optimal person compliance with taking medications resulting in the ineffective treatment and prolonged illness. The remains a need for alternative dosage forms for medications to treat various symptoms and illness.

US20160151279 A1 describes a semi-solid chewable dosage form that contains an active pharmaceutical ingredient, a gelling agent, gelatin, sugar, a polyol, and a pH adjusting agent.

### SUMMARY

The invention is defined by the claims.

In one aspect, the application provides methods for producing a gummy dosage form. The method includes the steps of providing a gelling blend, providing an active blend, combining the gelling blend and the active blend to provide a pre-molding blend, combing the pre-molding blend with a pH adjusting blend to provide a molding blend, and molding the molding blend to provide a gummy composition dosage.

In one embodiment, the providing a gelling blend comprises proving a water as a swelling solvent having a temperature from about 140F (60°C) to about 210F (98.9°C) and dissolving the pectin and the solubilizing blend in the water to provide a gelling blend. In one embodiment, a first bonding blend is additionally dissolved in the water. In one embodiment, an active blend is additionally dissolved in the water.

The combining step may comprise mixing the gelling blend with the emulsifying blend, followed by adding the second bonding blend to provide a pre-molding blend.

The active blend may be mixed in after the addition of the second bonding blend.

In one embodiment, the pre-molding blend is brought from a Brix number about 75 to a Brix number about 82 after addition of the second boding blend but before addition of the active blend.

The method may comprise providing a gelling blend comprising pectin, a solubilizing agent, water, optionally a sugar or a polyol; providing an active blend comprising an active agent; combining the gelling blend with the active blend to yield a matrix blend; combining the matrix blend with a bonding blend to provide a molding blend; and forming the molding blend into individual gummy dosage forms. The gelling blend may have a residual moisture content to between 5% by weight to 25% by weight.

### Gelling Blend

Pectin is a purified carbohydrate obtained by aqueous extraction from citrus peel or apple pomace. Any suitable type of pectin may be used including, for example, high-methoxy pectin and low-methoxy pectin and combinations thereof. Low-methoxy pectin may be amidated, which is often referred to as LMA pectin. Examples of suitable pectins are Genu.RTM. Citrus pectin USP/100 and Genu.RTM. citrus pectin USP/200 from CP Kelco.

Chemically, pectin molecule comprises methoxyl groups, carboxyl groups, and amide groups. The weight percentage of methoxyl group in the pectin molecule is termed the methoxyl content. The weight percentage of carboxyll group in the pectin molecule is termed the carboxyl content. The weight percentage of amide group in the pectin molecule is termed the amide content. According to the invention, the gelling blend comprises pectin having a carboxyl content of not more than 45% by weight of pectin and an amide content of not less than 20% by weight of pectin.

In one embodiment, the gelling blend comprises pectin having a methoxy content not less than 45% by weight of pectin. In one embodiment, the gelling blend comprises pectin having an amide content of not less than 25% by weight of pectin. In one embodiment, the gelling blend comprises pectin having a carboxyl content of less than 40% by weight of pectin.

In one embodiment, the pectin has a methoxy content of not less than 50%, 55%, 60%, 65%, 70%, 75%, 80%, 85%, 90% or 95%. In one embodiment, the pectin has a methoxyl content from about 45% to about 95% by weight of pectin. In one embodiment, the pectin has a methoxy content from about 55% to about 75% by weight of pectin. In one embodiment, the pectin has a methoxy content from about 55% to about 65% by weight of pectin.

In one embodiment, the pectin has an amide content of not less than 25%, 27%, of 30% by weight of the pectin. In one embodiment, the pectin has an amide content of not less than 32f%. 35, or 40% by weight of the pectin. In one embodiment, the pectin has an amide content of not less than 45% by weight of pectin. In one embodiment, the pectin has an amide content of not less than 50% by weight of pectin. In one embodiment, the pectin has an amide content from about 30% to about 40% by weight of pectin.

In one embodiment, the pectin has a carboxyl content of not more than 50%, 45%, 40%, 39%, 35%, 30%, 25%, or 20%. In one embodiment, the pectin has a carboxyl content not more than 40%.

In one embodiment, the pectin has a total of the carboxyl content and the amide content more than 50%, 55%, or 60%. In one embodiment, the pectin has a total of the carboxyl content and the amide content less than 80%, 75%, or 70%. In one embodiment, the pectin has a total of the carboxyl content and the amide content from about 56% to about 70%.

In one embodiment, the pectin comprises not more than 39% carboxyl groups and at least 27% amide groups. In one embodiment, the pectin comprises from 56 to 70% total amount of carboxyl group and amide groups. In one embodiment, the pectin comprises an amide content between 30 and 35%.

Through extensive research and testing, the surprise finding by the applicant is that the pectin with the property described herein combined with the methods of making disclosed herein produced a gummy composition dosage that is relatively heat stable when comparing to conventional gummy products. In one embodiment, the gummy composition has a melting point not less than 60°C. In one embodiment, the gummy composition has a melting point not less than 60°C. In one embodiment, the gummy composition has a melting point not less than 80°C. In one embodiment, the gummy composition has a melting point not less than 100C. In one embodiment, the gummy composition has a melting point not less than 200C. In certain experimentation in the applicant's lab, the gummy composition disclosed herein charred before melting.

Pectin may be present in the gummy composition dosage in an amount of from about 0.01% by weight to about 10% by weight. Pectin is present in an amount of from about 0.5% by weight to about 7% by weight, for example from about 0.5% to about 1%, from about 1% to about 1.5%, from about 1.5% to about 2%, from about 2% to about 2.5%, from about 2.5% to about 3%, from about 3% to about 3.5%, from about 3.5% to about 4%, from about 4% to about 4.5%, from about 4.5% to about 5%, from about 5% to about 5.5%, from about 5.5% to about 6%, from about 6% to about 6.5%, and from about 6.5% to about 7%. Pectin is present in an amount from about 1% by weight to about 5% by weight.

The swelling solvent may include any aprotic solvent. The swelling solvent may include water, an alcohol, or a combination thereof. The alcohol may include ethanol, glycerol, or a combination thereof. In one embodiment, the swelling solvent comprises water, or a combination of. water and glycerol.

The gelling blend may include pectin from about 5% to about 25% by weight. In one embodiment, the gelling blend comprises pectin from about 10% to about 20% by weight. In one embodiment, the gelling blend comprises pectin at about 14%.

Before the combining step, the gelling blend may have a Brix number from about 70 to about 95. In one embodiment, the gelling blend has a Brix number from about 75 to about 85. In one embodiment, the gelling blend has a Brix number of about 75.

Before the combining step, the gelling blend may be heated to from about 140°F (60°C) to about 240°F (115.6°C). In one embodiment, the gelling blend may be heated to from about 180°F (82.2°C) to about 210°F (98.9°C).

The application further provides a gelling blend, comprising pectin having a pectin content of at least 45%, a solubilizing blend, and optionally a bonding blend, wherein the solubilizing blend comprises a solubilizing salt having a formula of M-A, wherein M represent a cation of an alkaline or alkaline earth met According to the invention, the bonding blend includes having at least one pKa exceeding about 4. According to the invention, the bonding blend includes at least two of. a monosaccharide, a disaccharide and a trisaccharide. In one embodiment, the bonding blend comprises a flavoring agent, a coloring agent, an active agent, a preservative, or a combination thereof.

### Bonding Blend

According to the invention, the gelling blend includes a first bonding blend. A second bonding blend may be provided, and the second bonding blend may be combined with the gelling blend, the active blend, optionally emulsifying the blend to provide the pre-molding blend. According to the invention, the method comprises providing a second bonding blend, so that the first bonding blend and the second bonding blend form a bonding blend.

In one embodiment, the gelling blend may include a first bonding blend, and the first bonding blend and the second bonding blend as a whole form a bonding blend.

According to the invention, the bonding blend includes a combination of. at least two of. a monosaccharide, a disaccharide and a tri-saccharide.

In one embodiment, the bonding blend comprises a monosaccharide and a disaccharide. In one embodiment, the first bonding blend includes a monosaccharide and the second bonding blend includes a disaccharide. In one embodiment, the second blend comprises a monosaccharide and the first bonding blend includes a disaccharide. In one embodiment, both the first and the second bonding blend include a monosaccharide and a disaccharide.

In one embodiment, the monosaccharide and the disaccharide have a molar ratio from about 1:3 to about 3:1. In one embodiment, the monosaccharide and the disaccharide have a molar ratio from about 1:1.5 to about 1.5:1. In one embodiment, the monosaccharide and the disaccharide are in such a ration as to preventing the crystallization and co-crystallization of any of the saccharides in a solution having a Brix number from about 81 to about 90.

In one embodiment, bonding blend comprises a disaccharide and a tri-saccharide. In one embodiment, the bonding blend comprises a monosaccharide and a tri-saccharide. In one embodiment, the boding blend comprises a monosaccharide, a disaccharide and a tri-saccharide. The combination of varies saccharides can be achieved through various combination of the first and the second bonding blend. All variations are hereby incorporated in this disclosure.

In one embodiment, the monosaccharide comprises glucose, mannose, fructose, sorbose, tagatose, psicose, ribose, sorbose, galactose, xylose, lyxose, arabinose, ribulose, xylulose or a combination thereof. In one embodiment, the disaccharide comprises lactose, trehalose, palatinose, isomaltulose, maltose, cellobiose, chitobiose, sucrose, isomaltose, nigerose, kojibiose, sophorose, gentiobiose, mannobiose, rutinulose, turanose, melibiose, melibiulose, lactulose, or a combination thereof. In one embodiment, the tri-saccharide comprises raffinose, maltotriose, melezitose, isomaltotriose, kestose, nigertriose or a combination thereof.

In one embodiment, the bonding blend comprises a glucose syrup and sucrose. In one embodiment, the glucose syrup comprises corn syrup, tapioca syrup, rice syrup, barley syrup, cassava syrup, potato syrup or a combination thereof.

In one embodiment, the bonding blend includes tagatose and trehalose. In one embodiment, the bonding blend includes tagatose and palatinose. In one embodiment, the bonding blend includes psicose and trehalose. In one embodiment, the bonding blend includes psicose and palatinose. In one embodiment, the bonding blend includes psicose, trehalose and palatinose. In one embodiment, the bonding blend may include psicose, sorbose, trehalose, isomaltulose, or any combination thereof. The surprising discovery through the extensive testing and research by the applicant shows that any combination of tagatose, psicose, trehalost and palatinose (including a subset of at least two of these sugars) achieves a synergistically enhanced sweetness profile of the blend.

As the bonding blend may form a significant percentage of the gummy compositing, the bond blend may impart glycemic index to the gummy composition. In one embodiment, the bonding blend includes one low GI monosaccharide and one low GI disaccharide. The low GI saccharide refers to a saccharide having a GI value of not more than 30. In one embodiment, the low GI saccharide has a GI value of less than 12. In one embodiment, the gummy composition has a GI value of not more than 10, 12, 15, 18, 19, 20, 25, or 30. In one embodiment, the gummy composition is substantially free of starch.

### Solubilizing blend

The solubilizing blend is used to facilitate the swelling or solubilizing pectin. In one embodiment, the alkaline metal comprises Na, K, or a combination thereof. In one embodiment, the alkaline earth metal comprises Ca, Mg, or a combination thereof. In one embodiment, the multivalent acid has at least one pKa from about 5 to about 8. In one embodiment, the multivalent acid comprises citric acid, tartaric acid, galactic acid, malic acid. or a combination thereof

In one embodiment, the solubilizing salt and the pectin are present in a stoichiometric weight to weight ratio from about 1.4 to about 1:7 In one embodiment, the solubilizing salt and the pectin are present in a stoichiometric weight to weight ratio about 1.5

In one embodiment, the solubilizing salt and the pectin are present in such a stoichiometric weight to weight ratio such that from about 25% to about 75% of the carboxylic groups in pectin are configured to be deprotonated In one embodiment, the solubilizing salt and the pectin are present in such a stoichiometric weight to weight ratio such that from about 30% to about 60%of the carboxylic groups in pectin are configured to be deprotonated. In one embodiment, the solubilizing salt and the pectin are present in such a stoichiometric weight to weight ratio such that about 1/3 of the carboxylic groups in pectin are configured to be deprotonated.

### Emulsifying blend

An emulsifying blend may be combined with the gelling blend and the active blend to provide the pre-molding blend The emulsifying blend helps to facilitate the integration of various ingredients. In one embodiment, the emulsifying blend further helps to improve the texture or taste profile of the gummy composition dosage. In one embodiment, the emulsifying blend comprises a food grade emulsifier. In one embodiment, the emulsifying blend comprises a fatty acid or salt, oil, a gum, a peptide, a lipid or a combination thereof.

In one embodiment, the fatty acid comprises a saturated fatty acid, a unsaturated fatty acid, a salt or a derivative thereof. In one embodiment, the fatty acid comprises a carbon chain having from about 2 to about 22 carbons. In one embodiment, the fatty acid comprises oleic acid, linoleic acid, linolenic acid, palmitic acid, stearic acid, arachidonic acid, behenic acid, omega-3 fatty acid, docosahexaenoic acid, eicosapentaenoic acid, or a combination thereof the oil comprises coconut oil, palm oil, palm kernel oil, corn oil, rapeseed oil, grape seed oil, cannabis oil, sunflower oil, avocado oil, clary sage oil, olive oil, chia seed oil, flaxseed oil, linseed oil, fish oil, algae oil, krill oil or a combination hereof.

In one embodiment, the gum comprises acadia gum, xanthum gum, lotus seed gum, gum Arabic or a combination thereof.

In one embodiment, the peptide has a molecular weight not exceeding 10,000 Dalton In one embodiment, the peptide has a molecular weight from about 3000 to about 5000 Dalton In one embodiment, the lipid comprises wheat flour lipids, phospholipids, lecithin, or a combination thereof

### Active blend and active agent

The active blend may include an active agent and a liquid solvent In one embodiment, the active blend has a pH from about 5 5 to about 8.5 before the combining step. In one embodiment, the active blend has a pH from about 7.5 to about 8.

In one embodiment, the active blend may further include a neutralizing agent In one embodiment, the neutralizing agent and the active agent are present at approximately equivalent ratio stoichiometrically In one embodiment, the neutralizing agent comprises a basic agent.

In one embodiment, the basic agent comprises an inorganic salt, an organic base, an amino acid, or a combination thereof. The inorganic salt may include NaOH, Na2CO3, NaHCO3, KOH, KHCO3, K2CO3, Ca(HO3)2, CaCO3, Ca(OH)2, MgCO3, Mg(OH)2, C6H5Na3O7, C6H5K3O7, Mg2(C6H5O7)2, Ca2(C6H5O7)2 or a combination thereof In one embodiment, the organic base may be an organic compound having a primary or second amine group. In one embodiment, the organic base may be aspartame, glucosamine, caffeine, theobromine, xanthine, xanthine derivatives, nucleic acid base, or a combination thereof. In one embodiment, the amino acid comprises lysine, tryptophan, leucine, isoleucine, glutamine, glycine, methionine, cysteine, taurine, tyrosine, arginine, aspartic acid, tryptophan or a combination thereof.

The liquid solvent may include a diprotic solvent, tri-protic solvent, or a combination thereof The diprotic solvent may include water, propylene glycol, 1, 8-octanediol, 1,2-octanediol, 1,2-decanediol, 1,10-decanediol, or a combination thereof. The tri-protic solvent may include phosphoric acid, 1,2,3-propantriol, glycerol, glycerin, or a combination thereof. According to the invention, the active blend includes an active agent. The active agent may be any agent or compounds that can provide a biological benefit or therapeutic effect. In one embodiment, the active agent may be a nutritional or nutraceutical agent For example, the active agent may be collagen, a nutraceutical agent, a dietary supplement, a vitamin, an amino acid, an herbal extract or powder, a mineral, or a combination thereof.

In one embodiment, collagen useful as an active agent in the disclosed composition may have a MW from about 10K to about 50K Dalton. In one embodiment, the collagen has a MW of less than 50K Dalton. In one embodiment, the collagen has a MW of about 20K Dalton.

In one embodiment, the collagen and the pectin are present in a weight to weight ratio of about 1.4 in the gummy composition dosage In one embodiment, the collagen and the pectin are present in a weight to weight ratio of about 1:2 to about 1:8.

The active agent may be an API Example APIs include without limitation an antibacterial agent, an antifungal agent, a pain or fever reliever, an analgesics, an antiinflammatory agent a steroid, a diabetic medication, an antidiarrheal agent, an antiulcer agent, a proton pump inhibitor, a laxative or cathartic agent, a diuretic agent, an antacid, an antihistamine, a decongestant, an antitussive or expectorant agent, a cough suppressant, a cold medicine, a motion sickness medication, a medication for treating alcoholism or opioid dependence, a smoke cessation agent, an anti-anxiety agent, an antidepressant, a mood stabilizer, an antipsychotic agent, a stimulant, a benzodiazepine, an anxiolytic agent, a Z-drug, a melatonergic agent, a barbiturate, a antidepressant, an antipsychotic agent, a cardiovascular agent, an anti-cancer agent, an immune suppressant, a blood thinner, or a combination thereof

Representative antibacterial agent comprises penicillin, a cephalosporin, a macrolide, a fluroquinolone, a sulfonamide, a tetracycline, an aminoglycoside, or a combination thereof.

Representative antibacterial agent comprises bacitracin, clotrimazole, miconazole, penicillin, amoxicillin, cephalexin, erythromycin, clarithromycin, azithromycin, ciprofloxacin, levofloxacin, ofloxacin, co-trimoxazole, trimethoprim, tetracycline, doxycycline, gentamicin, tobramycin, or a combination thereof

Representative pain or fever reliever comprises acetaminophen, aspirin, salicylic acid, ibuprofen, naproxen, or a combination thereof

Representative antidiarrheal agent comprises loperamide, diphenoxylate bismuth subgallate, difenoxin, atropine, lactobacillus acidophilus, saccharomyces boulardii lyo, attapulgite, its derivative thereof, or a combination thereof.

Representative antiulcer agent comprises cimetidine, famotidine, nizatidine, rantidine, misoprostol, sucralfate or a combination thereof.

Representative proton pump inhibitor comprises omeprazole, esomeprazole, rabeprazole, pantoprazole, lansoprazole, dexlansoprazole or a combination thereof

Representative laxative or cathartic agent comprises bisacodyl, docusate, miralax, psyllium, senokot, castor oil, magnesium hydroxide, magnesium citrate, magnesium sulfate, lactulose or a combination thereof.

Representative antihistamine comprises cetirizine, diphenhydramine, loratadine, chlorpheniramine, brompheniramine, alimemazine, cyprohetadine, doxylamine, hydroxyzine, promethazine, or a combination thereof.

Representative decongestant comprises pseudoephedrine, oxymetazoline, phenylephrine or a combination thereof.

Representative antitussive and expectorant comprises guaifenesin, codeine phosphate, dextromethorphan hydrobromide, or a combination thereof.

Representative cough suppressant comprises dextromethorphan.

Representative medication for treating alcoholism or opioid dependence comprises acamprosate, baclofen, buprenorphine, naloxone, clonidine, disulfiram, methadone, naltrexone, ondansetron, or a combination thereof.

Representative smoke cessation agent comprises nicotine, bupropion, cytosine, varenicline, or a combination thereof.

Representative mood stabilizer comprises carbamazepine, gabapentin, lamotrigine, levetiracetam, lithium salt, oxcarbazepine, topiramate, sodium valproate, divalproex sodium, valproic acid,

Representative antipsychotic agent comprises aripiprazole, asenapine, chlorpromazine, olanzapine, paliperidone, quetiapine, risperidone, ziprasidone,

Representative stimulant comprises amphetamine, dexamfetamine, lisdexamfetamine, methylphenidate, dexmethylphenidate, metamfetamine, or a combination thereof.

Representative benzodiazepine comprises alprazolam, bromazepam, chlordiazepoxide, clobazam, clonazepam, clorazepate, diazepam, lorazepam, oxazepam, tofisopam, brotizolam, estazolam, flunitrazepam, flurazepam, loprazolam, lormetazepam, midazolam, nimetazepam, nitrazepam, phenazepam, quazepam, temazepam, triazolam, or a combination thereof.

Representative anxiolytic agent comprising buspirone, hydroxyzine, meprobamate, pregabalin, or a combination thereof.

Representative Z-drug comprises eszopicolone, zaleplon, zolpidem, zopiclone, or a combination thereof.

Representative melatonergic agent comprises agomelatine, ramelteon, or a combination thereof.

Representative barbiturate comprises amobarbital, secobarbital, butobarbital, cyclobarbital, diazepam, pentobarbital, phenobarbital, secobarbital, or a combination thereof.

Representative antidepressant comprises citalopram, clomipramine, dexopin, escitalopram, fluoxetine, fluvoxamine, imipramine, mirtazapine, paroxetine, sertraline, trazodone, amitriptyline, doxepin, mianserin, mirtazapine, trazodone, trimipramine, or a combination thereof.

Representative antipsychotic agent comprises chlorprothizene, levomepromazine, perazine, promethazine, prothipendyl, sulpiride, thioridazine, zuclopenthixol, perphenazine, beneperidol, bromperidol, fluphenazine, fluspirilen, haloperidol, pimozide, amisulpride, aripiprazole, asenapine, chloropromazine, clozapine, flupenthixol, lloperidone, melperone, olanzapine, paliperidone, penfluridol, quetiapine, reserpine, risperidone, sertindole, thiothizene, trifluoperazine, zipraisdone, zotepine, pericyazine, carbamazepine, valproic acid, or a combination thereof.

Representative cardiovascular agent comprises an aldosterone receptor antagonist, an angiotensin converting enzyme inhibitor, an angiotensin receptor blocker, a neprilysin inhibitor, an antiadrenergic agent, an antianginal agent, an antiarrhythmic agent, an anticholinergic chronotropic agent, an antihypertension agent, an ACE inhibitor, an angiotensin 11 inhibitor, an antiadrenergic agent, a beta blocker, a diuretic agent, a beta-adrenergic blocker, a calcium channel blocker, a catecholamine, an inotropic agent, a vasodilator, a renin inhibitor, a sclerosing agent, a vasopressin antagonist, a vasopressor, an anti-cholesterol agent, or a combination thereof.

Representative antihypertension agent comprises diazoxide, fenoldopam, nitroprusside, a thiazide, or a combination thereof.

In one embodiment, the active agent includes caffeine, aspirin, acetaminophen, ibuprofen, diphenhydramine, naproxen, a PDE5 inhibitor, of a derivative thereof. Example PDES inhibitor include avanafil, iodenafil, mirodenafil, udenafil, zaprinast, icariin, benzamidenafil, dasantafil, sildenafil, tadalafil, vardenafil hydrochloride, or a derivative or combination thereof.

The active agent may include at least one functional group having a pKa from about 2 to about 9. In one embodiment, the active agent may include one functional group having a pKa from about 3 to about 5 In one embodiment, the functional group may include a carboxylic group. In one embodiment, the functional group may include a phenol group.

In one embodiment, the active agent may include aspirin, salicylic acid, penicillin, fenoprofen, moxifloxacin, cromolyn salt, or a derivative thereof. In one embodiment, the active agent may include sulfamethoxazole, phenol barbital, phenytoin, chlorthalidone, vidagliptin, phenobarbital, furosemide, tetracycline, or a derivative thereof. In one embodiment, the active agent may include aspirin and caffeine. In one embodiment, the active agent may include aspirin and caffeine having a molar ratio from about 15:1 to about 100:1.

### pH adjusting blend

The pH adjusting blend functions to adjust the pH of the molding blend. According to the invention, the molding blend has a pH value at least about 5 The molding blend may have
a pH value at least about 4.5. The molding blend may have a pH value at least about 3, 3.5, or 4.

In one embodiment, the pH adjusting blend comprises a pH adjusting acid In one embodiment, the pH adjusting acid comprises a multivalent acid. The multivalent acid may include without limitation citric acid, tartaric acid, galactic acid, malic acid, phosphoric acid, or a combination thereof. In one embodiment, the pH adjusting acid is configured to re-protonate at least a porting of carboxylic groups in the pectin.

### Pre-molding blend and molding blend

In one embodiment, the pre-molding blend has a Brix number from about 70 to about 80 In one embodiment, the pre-molding blend has a Brix number about 75. In one embodiment, the pre-molding blend has a temperature from about 140F to about 250F.

In one embodiment, the molding blend has a higher Brix number than the pre-molding blend. In one embodiment, the molding blend has a Brix number from about 80 to about 90. In one embodiment, the molding blend has a Brix number from about 81 to about 84. In one embodiment, the molding blend has a Brix number from about 82 to about 83.

In one embodiment, the molding blend comprises the pectin at a concentration from about 7% to about 10% by weight. In one embodiment, the molding blend comprises the pectin at a concentration of about 8%.

In one embodiment, the molding blend is a flowable liquid. In one embodiment, the molding solution achieves a viscosity of from about 250 cP (0.25 Pa s) to about 6000 cP (6 Pa·s) before the forming step.

### Molding blend

In one embodiment, the molding blend sets into a gummy composition in less than 15 minutes. In one embodiment, the molding blend sets into a gummy composition in less than 5 minutes. In one embodiment, the molding blend sets into a gummy composition in about 3 minutes. In one embodiment, the forming the molding blend comprises injecting the molding blend into a mold and forming the molding blend into a gummy composition.

### Gummy composition dosage

The gummy composition dosage may have a weight from about 2grams to about 8 grams per piece. In one embodiment, the gummy composition dosage has a weight from about 3grams to about 5 grams. In one embodiment, the gummy composition dosage has a weight of about 5 grams. In one embodiment, the gummy composition dosage has a weight of about 7.5 grams.

The gummy composition dosage may include from about 1mg to about 500mg of the active agent. In one embodiment, the gummy composition dosage comprises about 81mg of aspirin or its derivative thereof. In one embodiment, the gummy composition dosage comprises about 325mg of aspirin or its derivative thereof. In one embodiment, the gummy composition dosage may include about 10mg of cetirizine. In one embodiment, the gummy composition dosage may include about 10mg of loratadine. In one embodiment, the gummy composition dosage may include about 12 mg of diphenhydramine. In one embodiment, the gummy composition dosage may include from about 50 mg of ibuprofen. In one embodiment, the gummy composition dosage may include from about 125 mg of acetaminophen.

The gummy composition dosage may be any shape. Example shapes include without limitation hexagon, disk, column, ball, gum drop, square, cube, animal shape, hear shape, figurine shaped, letter shaped, donut shaped, fruit shaped, or a combination thereof.

In another aspect, the disclosure provides pectin based gummy composition that contains an active agent. The active agent could be a nutraceutical agent or a pharmaceutical agent. In one embodiment, the active agent may be vitamins, minerals, natural products, herbal extract, herbal powders, amino acids, lipids, fatty acids. In one embodiment, the gummy composition is a dietary or nutritional supplement.

In one embodiment, the active agent may be an active pharmaceutical ingredient (API) or a bioactive ingredient. The pharmaceutical agent may be small molecule compound or a biologics. The pharmaceutical agent may be hydrophilic or lipophilic. In one embodiment, the active agent comprises large, hydrophilic and unstable proteins, oligonucleotides and polysaccharides, as well as conventional small drug molecules.

In one embodiment, the gummy composition is an OTC drug. In one embodiment, the gummy composition is a legend drug. In one embodiment, the gummy composition is a prescription drug.

In one embodiment, the gummy composition further includes a flavoring agent, a coloring agent, an active agent, a preservative, or a combination thereof.

In one instance, the application provides a medication kit including a gummy composition dosage disclosed herein. The medication kit may further include an instruction for using such gummy composition. According to the invention, there is provided a medication kit comprising a gummy composition of the invention and an instruction for using such gummy composition for treating a disease.

In a further instance, the application provides methods of making a gummy composition using a low methoxy content pectin. In one instance, the method includes the step of providing a gelling blending, wherein the gelling blend comprises pectin having a methoxy content of less than 45% and a swelling blend; providing an active blend, wherein the active blend comprises an active agent; providing a bonding blend, wherein the bonding blend comprises a multivalent cation; combining the gelling blend and the active blend to a first blend having a Brix number from about 75 to about 85; adding the bonding blend to the first blend to provide a molding blend; and forming the molding blend into a gummy composition.

In one instance, the method further includes adding a flavoring blend into the first blend before combining the gelling blend with the bonding blend. In one instance, the molding blend comprises the bonding blend at a concentration from about 0.1% to about 1% by weight In one instance, the molding blend comprises the bonding blend at a concentration at about 0.5% by weight.

In one instance, the multivalent cation comprises the cation of calcium, magnesium, strontium, barium, copper, chromium, manganese, cobalt, zinc, nickel, gallium, aluminum, or a combination thereof.

In a further instance, the disclosure provides methods for delivering an active agent through oral mucosal delivery route. The disclosure provides gummy compositions and dosages that are configured to be consumed by chewing therefore delivering the active agent through oral mucosal absorption. Through extensive research and by adjusting the ratio between the gelatin blend, the bonding blend, and optionally plasticizing blend, as well as the MW of the gelling and/or bonding agent, the gummy composition with controlled melting and chewing time can be achieved. In one embodiment, the gummy composition is configured to melt in an oral cavity therefore provide a chewing time not less than 20 seconds, 30 seconds, 1, 2, 3, 4, 5, 6, 7, 8, 9, or 10 minutes. In one embodiment, the gummy composition is gum like and may provide a chewing time of not less than 5, 7 or 10 minutes.

The oral mucosal drug delivery may have significant advantage of conventional routes of administration. See, for example, A Detailed Review on Oral Musocal Drug Delivery System, International Journal of Pharmaceutical Sciences and Research, ICV (2015): 90.24; ISSN (Online): 0975-8232, ISSN (Print): 2320-5148.

Oral mucosal drug delivery is an alternative method of systemic drug delivery that offers several advantages over both injectable and enteral methods. Because the oral mucosa is highly vascularized, drugs that are absorbed through the oral mucosa directly enter the systemic circulation, bypassing the gastrointestinal tract and first-pass metabolism in the liver. For some drugs, this results in rapid onset of action via a more comfortable and convenient delivery route than the intravenous route.

Examples cardiovascular drugs that can be administered trans-mucosally through the disclosed gummy formulation include nitroglycerin, captopril, verapamil and propafenone, has proven promising. Examples of trans-mucosal delivery of analgesics using the present gummy formulation may include fentanyl, which may be deliver rapid analgesia for breakthrough pain, providing patients with a noninvasive, easy to use and nonintimidating option. Examples of oral mucosal delivery of sedatives using the present gummy formulation may include midazolam, triazolam and etomidate. Examples of oral mucosal delivery of the anti-nausea drugs using the present gummy formulation may include scopolamine and prochlorperazine. Examples of oral mucosal delivery of drugs may also include compounds for treating erectile dysfunction. Oral trans-mucosal formulations of testosterone and estrogen have been developed. In clinical studies, sublingual testosterone has been shown to result in increases in lean muscle mass and muscle strength, improvement in positive mood parameters, and increases in genital responsiveness in women. Short-term administration of estrogen to menopausal women with cardiovascular disease has been shown to produce coronary and peripheral vasodilation, reduction of vascular resistance and improvement in endothelial function.

The small molecule compound may be sufficiently small whereby the compound may pass the oral mucosal membrane and enter the blood stream during the gummy chewing process. In some embodiments, the molecule weight of the active compound is from about 50 Dalton to about 500 Dalton. In some embodiment, the molecule weight is from about 100 Dalton to about 250 Dalton. The small molecule compound may be an acidic compound, a basic compound, or a zwitterion compound.

The active agent may be asenapine, chlorpheniramine maleate, phenylephrine hydrochloride, guaifenesin, dextromethorphan hydrobromide, loratadine, diphenhydramine, or a combination thereof. In one embodiment, the active agent useful for treating gastrointestinal disorders may be an antacid, an anti-foaming agent, a histamine H2-receptor antagonist (commonly known as a H2 antagonist), proton pump inhibitor, or a combination thereof. Other active agents commonly present in OTC medications or legend medications are suitable for use in the present disclosure.

In one instance, the application provides methods for treating a disease comprising the step of administering an effective amount of herein disclosed gummy composition dosage to a subject to prevent, reduce, or treat a disease. The gummy dosage form according to the disclosure is useful for administration to individuals, including both adults and children as dietary supplement or pharmaceuticals for treating symptoms including without limitation allergies, motion sickness, colds, coughs, pains, fever, gastrointestinal disorders, sleep, neurological disorders, cardiovascular disorders, and lung diseases.

In another instance, the method for using the pectin based gummy has been disclosed. The gummy may be used tor treating or controlling various diseases, improving health, supplementing diet, or improving athletic performance.

Other embodiments, characteristics, and advantages of the disclosure are apparent after reading the descriptions and examples that follow.

### DETAILED DESCRIPTION

The gummy dosage form of the disclosure is intended to be chewed by a person such that it is broken up into smaller parts or melt within the oral cavity. The active agent may be released from the gummy dosage and absorbed efficiently through mucosal membrane into blood stream allowing a quick release and fast absorption of the active ingredient. In addition, the smaller pieces may be easily swallowed thereby providing an administration advantage over traditional tablets based dosage forms.

The gummy dosage form may have a sufficiently high viscosity that it is not pourable and further does not flow or conform to its container at room temperature. Typically, the gummy dosage form does not flow at low shear stress and generally exhibits plastic flow behavior. In general, the consistency of the gummy dosage form is the same as or similar to gelatin-based or pectin-based products.

The dosage form can have any size and shape such that it can be administered orally and chewed by a person. The person should be able to readily break apart the dosage form by chewing and swallow the dosage form without the need for an external source of liquid. Typically, the dosage form has a length of about 1 cm to about 5 cm, width of about 1 cm to about 5 cm and a height of about 1 cm to about 5 cm. Suitable shapes include, for example, ovals, spheres, cylinders, hemisphere, hexagon, rectangular boxes, and cubes. The dosage form may be formed into unique shapes and figures including, for example, animals for administration to children (e.g., under the age of 13) and/or adults.

In one embodiment, each individual dosage form has a total weight of at least 100 mg. In some embodiments, each dosage form has a total weight of from about 1 g to about 20 g. , each dosage form has a total weight of from about 1 g to about 15 g. each dosage form has a total weight of from about 1 g to about 10 g, for example, about 1 g to about 1.5 g, about 1.5 g to about 2 g, about 2 g to about 2.5 g, about 2.5 g to about 3 g, about 3.5 g to about 4 g, about 4 g to about 4.5 g, about 4.5 g to about 5 g, about 5 g to about 5.5 g, about 5.5 g to about 6 g, about 6 g to about 6.5 g, about 6.5 g to about 7 g, about 7 g to about 7.5 g, about 7.5 g to about 8 g, about 8 g to about 8.5 g, about 8.5 g to about 9 g, about 9 g to about 9.5 g, and about 9.5 g to about 10 g. Each dosage form has a total weight of about 5 g.

Active agents that are suitable for use in the gummy dosage form for the disclosure include, by way of example, anti-allergy, antihistamines, antitussives, decongestants, expectorants, anti-cold/flu, analgesics, anti-inflammatories, sleep medications, anti-heartburn medications, anti-gas medications, anti-GERD medications, anti-diarrheas, laxatives, antismoking and/or motion sickness medications. In some embodiments, suitable active agents may be suitable for treating and/or preventing gastrointestinal disorders including, for example, antacids, anti-foaming agents, H2 antagonists, proton pump inhibitors, anti-diarrheas, laxatives, or a combination thereof. Suitable active agents useful in the gummy dosage form of the disclosure are typically available as over-the-counter medications.

The gummy dosage of the disclosure is further useful for administration to individuals, including both adults and children, to treat and/or prevent allergies, colds and coughs, as well as symptoms of these conditions. Additionally, the gummy dosage form of the disclosure may be used to treat or prevent gastrointestinal disorders and symptoms thereof such as dyspepsia, peptic ulcer, gastroesophageal reflux disease, upset stomach, heartburn, excessive gas, and the like along with symptoms of these disorders.

In other embodiments, the gummy dosage form of the disclosure includes one or more active agents useful for the treatment of gastrointestinal disorders and symptoms thereof. Such active ingredients are typically available as over-the-counter medications.

Any suitable active agents may be used in the gummy dosage form of the present disclosure to treat or prevent one or more symptoms. Example active agents include without limitation, anti-allergy agents such as loratadine, diphenhydramine, cetirizine and fexofenadine; anti-cold or flu agents such as pseudoephedrine, phenylephrine and and chlorpheniramine; analgesics such as ibuprofen, aspirin, naproxen, acetaminophen, and codeine; cough medications such as guaifenesin and dextromethorphan; sleep medications such as diphenhydramine and doxylamine; heartburn medications such as ranitidine, cimetidine, famotidine, omeprazole, esomeprazole, lansoprazole, calcium carbonate and bismuth subsalicylate; anti-diarrheas such as loperamide; anti-gas agents such as simethicone; laxatives such as bisacodyl; smoking cessation agents such as nicotine, motion sickness medications such as dimenhydrinate meclizine.

Combinations of two or more active agents may be used in the gummy dosage form of the disclosure to treat or prevent one or more symptoms. Suitable combinations of active agents include for example: anti-allergy combinations such as loratadine/ pseudoephedrine, diphenhydramine/ phenylephrine, diphenhydramine/ pseudoephedrine, cetirizine/ pseudoephedrine, and fexofenadine/pseudoephedrine; anti-cold and flu combinations such as chlorpheniramine/ phenylephrine, ibuprofen/ phenylephrine, dextromethorphan/ ibuprofen/phenylephrine, dextromethorphan/ acetaminophen/ phenylephrine, acetaminophen/ phenylephrine, acetaminophen/ pseudoephedrine, doxylamine/ dextromethorphan/ acetaminophen, acetaminophen/ dextromethorphan/ guaifenesin/ phenylephrine; analgesics combinations such as ibuprofen/ caffeine, aspirin/ caffeine, naproxen/ caffeine, acetaminophen/ caffeine, diphenhydramine/ ibuprofen, diphenhydramine/ acetaminophen, diphenhydramine/ naproxen, acetaminophen/ caffeine/ aspirin; and antitussive combinations such as guaifenesin/ dextromethorphan; sleep medication combinations such as diphenhydramine/ ibuprofen, diphenhydramine/ aspirin, diphenhydramine/ naproxen, diphenhydramine/ acetaminophen; anti-gas combination such as simethicone/calcium carbonate.

The active agents used in the gummy dosage form of the disclosure include chlorpheniramine maleate, phenylephrine hydrochloride, guaifenesin, dextromethorphan hydrobromide, loratadine, diphenhydramine, or a combination thereof. In one embodiment, the dosage form contains a combination of chlorpheniramine maleate and phenylephrine hydrochloride. In another embodiment, the dosage form contains a combination of dextromethorphan hydrobromide and phenylephrine hydrochloride. Chlorpheniramine maleate is a pharmaceutically acceptable salt of chlorpheniramine.

In some embodiments, the amount of chlorpheniramine maleate present in each dosage form is from about 0.1 mg to about 30 mg. The amount of chlorpheniramine maleate present in each dosage form is from about 1 mg to about 10 mg. The amount of chlorpheniramine maleate present in each dosage form is about 1 mg to about 5 mg. The amount of chlorpheniramine present is about 2 mg or about 4 mg in each dosage form that has a total weight of about 5 g.

Chlorpheniramine maleate may be present in the dosage form in amount from about 0.01% by weight to about 1.0% by weight, and about 0.02% to about 0.2% by weight. For an adult dose, chlorpheniramine maleate is present in an amount from about 0.06% by weight to about 0.1% by weight. For a pediatric dose (e.g., children under 13), chlorpheniramine maleate is present in an amount from about 0.03% by weight to about 0.05% by weight.

In some embodiments, the amount of phenylephrine hydrochloride present in each dosage form is from about 0.1 mg to about 20 mg. The amount of phenylephrine hydrochloride present is from about 2 mg to about 15 mg. The amount of phenylephrine hydrochloride present is from about 3 mg to about 12 mg. The amount of phenylephrine hydrochloride present is about 5 mg or about 10 mg in each dosage form that has a total weight of about 5 g.

Phenylephrine hydrochloride may be present in the dosage form in amount from about 0.01% by weight to about 1% by weight, and 0.01% to about 0.5% by weight. For an adult dose, phenylephrine hydrochloride is present in an amount from about 0.15% by weight to about 0.25% by weight. For a pediatric dose (e.g., children under 13), phenylephrine hydrochloride is present in an amount from about 0.05% by weight to about 0.15% by weight.

In some embodiments, the amount of guaifenesin present in each dosage form is from about 10 mg to about 1,500 mg. The amount of guaifenesin present in each dosage form is from about 200 mg to about 1,200 mg. The amount of guaifenesin present in each dosage form is about 100 mg to about 400 mg. The amount of guaifenesin present is about 100 mg, 200 mg or about 400 mg in each dosage form that has a total weight of about 5 g.

Guaifenesin may be present in the dosage form in amount from about 0.1% by weight to about 20% by weight, and about 0.5% to about 10% by weight. For an adult dose, guaifenesin is present in an amount from about 0.5% by weight to about 5% by weight. For a pediatric dose (e.g., children under 13), guaifenesin is present in an amount from about 0.1% by weight to about 4% by weight.

In some embodiments, the amount of dextromethorphan hydrobromide present in each dosage form is from about 1 mg to about 100 mg. The amount of guaifenesin present in each dosage form is from about 5 mg to about 60 mg. The amount of guaifenesin present in each dosage form is about 10 mg to about 30 mg. The amount of guaifenesin present is about 10 mg or about 20 mg in each dosage form that has a total weight of about 5 g.

Dextromethorphan hydrobromide may be present in the dosage form in amount from about 0.01% by weight to about 2% by weight, and about 0.1% to about 1% by weight. For an adult dose, guaifenesin is present in an amount from about 0.1% by weight to about 1% by weight. For a pediatric dose (e.g., children under 13), guaifenesin is present in an amount from about 0.1% by weight to about 0.8% by weight.

In some embodiments, the amount of loratadine present in each dosage form is from 1 mg to about 100 mg. The amount of loratadine present in each dosage form is from about 5 mg to about 50 mg. The amount of loratadine present in each dosage form is from about 10 mg to about 30 mg. The amount of loratadine present is about 10 mg in each dosage form that has a total weight of about 5 g.

Loratadine may be present in the dosage form in amount from about 0.01% by weight to about 2% by weight, and about 0.1% to about 1% by weight. For an adult dose, loratadine is present in an amount from about 0.1% by weight to about 1% by weight. For a pediatric dose (e.g., children under 13), loratadine is present in an amount from about 0.1% by weight to about 0.5% by weight.

In some embodiments, the amount of diphenhydramine hydrochloride present in each dosage form is from 1 mg to about 100 mg. The amount of diphenhydramine hydrochloride present in each dosage form is from about 5 mg to about 50 mg. The amount of diphenhydramine hydrochloride present in each dosage form is from about 10 mg to about 30 mg. The amount of diphenhydramine hydrochloride present is about 12.5 mg or 25 mg.

Diphenhydramine hydrochloride may be present in the dosage form in amount from about 0.01% by weight to about 2% by weight, and about 0.1% to about 1% by weight. For an adult dose, diphenhydramine hydrochloride is present in an amount from about 0.1% by weight to about 1% by weight. For a pediatric dose (e.g., children under 13), diphenhydramine hydrochloride is present in an amount from about 0.1% by weight to about 0.5% by weight.

Chlorpheniramine maleate and phenylephrine hydrochloride may optionally both be present in combination in the dosage form. , in such embodiments, chlorpheniramine maleate is present in an amount of about 2 mg and phenylephrine hydrochloride is present in an amount of about 5 mg. In one embodiment, chlorpheniramine maleate is present in an amount of about 4 mg and phenylephrine hydrochloride is present in an amount of about 10 mg. Typically, a pediatric dose contains about 2 mg chlorpheniramine maleate and 5 mg phenylephrine hydrochloride and an adult dose contains about 4 mg chlorpheniramine maleate and 10 mg phenylephrine hydrochloride.

In embodiments, dextromethorphan hydrobromide and phenylephrine hydrochloride are both present in the dosage form. Dextromethorphan hydrobromide is present in an amount of about 10 mg and phenylephrine hydrochloride is present in an amount of about 5 mg. Dextromethorphan hydrobromide is present in an amount of about 20 mg and phenylephrine hydrochloride is present in an amount of about 10 mg.

In other embodiments, the active agent is an antacid, anti-foaming agent, histamine H2-antagonist, proton pump inhibitor, anti-diarrheal, laxative, antibiotics, anti-fungal, anti-diabetes, anti-cancer, or combination thereof, that are useful for the treatment and/or prevention of gastrointestinal disorders or symptoms thereof.

Suitable antacids including, but are not limited to, potassium bicarbonate, sodium bicarbonate, calcium bicarbonate, aluminum bicarbonate, magnesium bicarbonate, magnesium hydroxide, calcium carbonate, aluminum hydroxide, and combinations thereof.

In one embodiment, the antacid is calcium carbonate. Calcium carbonate has the formula Ca2CO3. The calcium carbonate can be anhydrous calcium carbonate or a hydrate thereof. Suitable histamine H2-receptor antagonists include, but are not limited to, cimetidine, ranitidine, famotidine, and nizatidine. In one embodiment, the histamine H2-receptor antagonist is famotidine. Suitable proton pump inhibitors include, but are not limited to, omeprazole, lansoprazole, dexlansoprazole, esomeprazole, pantoprazole, rabeprazole, and ilaprazole. In an embodiment, the proton pump inhibitor is omeprazole. A suitable anti-diarrheal includes, but is not limited to, loperamide. A suitable laxative includes, but is not limited to, bisacodyl. A suitable anti-gas agent includes, but is not limited to, simethicone.

The amount of active agent in the gummy dosage form will vary for each different active depending on its use. The amount present will usually be less for gummy dosage forms that are to be administered to children. For gummy dosage forms for use with gastrointestinal disorders, the active ingredient may be present in an amount sufficient to treat and/or prevent gastrointestinal disorders and symptoms thereof (e.g., bloating, discomfort and/or pain) including, for example, dyspepsia, peptic ulcer, gastroesophageal reflux disease, upset stomach, heartburn, and excessive gas. Typically, the gummy dosage form contains about 0.1 mg to 1 g of the active agent. In one embodiment, the gummy dosage form contains one or more active agents in an amount from about 0.01% by weight to about 10% by weight.

The gummy dosage form of the disclosure may be administered once per day or multiple times per day to provide relief for various symptoms affecting an individual. For example, chlorpheniramine maleate may be administered to treat symptoms of allergic rhinitis or sinusitis. Phenylephrine hydrochloride may be administered to treat symptoms of nasal congestion. Typical dosing of chlorpheniramine maleate for adults is 4 mg every 4-6 hours and for children (i.e., 6-11 years old) is 2 mg every 4-6 hours. Typical dosing of phenylephrine hydrochloride for adults is 10 mg every 4-6 hours and for children (i.e., 6-11 years old) is 5 mg every 4-6 hours.

Aspirin may be administered to treat symptoms of fever, pain or for cardiovascular health. Typical dosing of aspirin for adults is 81mg for cardiovascular health and 325mg for pain or fever relief and for children (i.e., 6-11 years old) is 81mg.

Naproxen may be administered to relieve pain. Typical dosing of naproxen for adults is 275mg to 500mg and for children (i.e., 6-11 years old) is 250mg or less.

Acetaminophen may be administered to relieve pain or fever. Typical dosing of acetaminophen for adults is 325mg to 650mg and for children (i.e., 6-11 years old) is 10-15mg/Kg.

Guaifenesin may be administered to treat symptoms of congestion in the chest and throat. Typical dosing of guaifenesin for adults is 200 mg to 400 mg every 4-6 hours and for children (i.e., 6-11 years old) is 100 mg to 200 mg every 4-6 hours.

Dextromethorphan hydrobromide may be administered to treat symptoms of a cough. Typical dosing of dextromethorphan hydrobromide for adults is 10 mg to 30 mg every 4-8 hours and for children (i.e., 6-11 years old) is 5 mg to 10 mg every 4 hours.

Loratadine, cetirizine, or diphenhydramine may be administered to treat symptoms of allergic rhinitis and urticaria. Typical dosing of loratadine for adults and children (i.e., 6-11 years old) is 10 mg per day. Typical dosing of cetirizine for adults and children (i.e., 6-11 years old) is 10 mg per day. Typical dosing of diphenhydramine for adults and children (i.e., 6-11 years old) is 12.5mg or 25 mg per day.

In embodiments where the active agent is an antacid, the amount of antacid present in each dosage form is from about 10 mg to about 2 g. The amount of antacid present in each dosage form is from about 100 mg to about 1 g. The amount of antacid present in each dosage form is about 500 mg to about 1 mg. The amount of antacid present is about 750 mg or about 800 mg in each dosage form that has a total weight of about 5 g. The antacid may be present in the dosage form in amount from about 1% by weight to about 30% by weight, and about 5% to about 20% by weight.

In embodiments where the active agent is an anti-foaming agent (also referred to herein as an anti-gas agent), the amount of anti-foaming agent present in each dosage form is from about 1 mg to about 500 mg. The amount of anti-foaming agent present is from about 5 mg to about 250 mg. The amount of anti-foaming agent present is from about 10 mg to about 100 mg. The amount of anti-foaming agent present is about 20 mg or about 80 mg in each dosage form that has a total weight of about 5 g.

The anti-foaming agent may be present in the dosage form in amount from about 0.01% by weight to about 5% by weight, and 0.1% to about 5% by weight. For an adult dose, the anti-foaming agent is present in an amount from about 0.15% by weight to about 0.25% by weight. For a pediatric dose (e.g., children under 13), the anti-foaming agent is present in an amount from about 0.05% by weight to about 0.15% by weight.

In some embodiments, the active agent is a histamine H2-receptor antagonist. In these embodiments, the amount of histamine H2-receptor antagonist present in each dosage form is from about 1 mg to about 500 mg. , the amount of histamine H2-receptor antagonist is from about 5 mg to about 250 mg. The amount of histamine H2-receptor antagonist present is from about 10 mg to about 100 mg. The amount of histamine H2-receptor antagonist present is about 20 mg or about 80 mg in each dosage form that has a total weight of about 5 g.

In some embodiments, the active agent is a proton pump inhibitor. In these embodiments, the amount of proton pump inhibitor present in each dosage form is from about 1 mg to about 500 mg. The amount of proton pump inhibitor is from about 5 mg to about 250 mg. The amount of proton pump inhibitor present is from about 10 mg to about 100 mg. The amount of proton pump inhibitor present is about 20 mg or about 80 mg in each dosage form that has a total weight of about 5 g.

The gummy dosage form of the disclosure includes a gelling agent. Any suitable gelling agent may be used to provide the dosage form with the desired characteristics including, for example, gummy structure, shape and texture. The gelling agent is typically a USP (U.S. Pharmacopeia) grade gelling agent. The gelling agent may be pectin.

Pectin is a purified carbohydrate obtained by aqueous extraction from citrus peel or apple pomace. Any suitable type of pectin may be use in the dosage form including, for example, high-methoxy pectin and low-methoxy pectin and combinations thereof. Low-methoxy pectin may be amidated, which is often referred to as LMA pectin. Examples of suitable pectins are Genu.RTM. citrus pectin USP/100 and Genu.RTM. citrus pectin USP/200 from CP Kelco.

Pectin may be generally present in the gummy dosage form in an amount of from about 0.01% by weight to about 10% by weight. Pectin is present in an amount of from about 0.5% by weight to about 7% by weight, for example from about 0.5% to about 1%, from about 1% to about 1.5%, from about 1.5% to about 2%, from about 2% to about 2.5%, from about 2.5% to about 3%, from about 3% to about 3.5%, from about 3.5% to about 4%, from about 4% to about 4.5%, from about 4.5% to about 5%, from about 5% to about 5.5%, from about 5.5% to about 6%, from about 6% to about 6.5%, and from about 6.5% to about 7%. Pectin is present in an amount from about 1% by weight to about 5% by weight.

In some embodiments, the gummy dosage form of the disclosure may further include gelatin. Without being bound by any theory and through extensive testing and research, the applicants discovered that that the presence of gelatin in combination with pectin and the methods of making disclosed herein assists with gelling of the gummy composition and further serves to mask or reduce the taste of the active ingredients.

Any suitable type of gelatin may be present in the dosage form. For example, the gelatin may be animal-derived gelatin, chemically-modified gelatin, physically-modified gelatin, and combinations thereof. Animal-derived gelatin may be derived from any suitable source such as, for example, pigskin or bovine bone.

In one embodiment, the gelatin may be hydrolyzed gelatin. Hydrolyzed gelatin is also commonly known as hydrolyzed collagen, collagen hydrolysate, and collagen peptide. Hydrolyzed gelatin having a molecular weight ranging from about 2,500 to about 5,000 may be used. An example of a suitable hydrolyzed gelatin is Peptiplus.RTM. powder from Gelita.

Gelatin may be generally present in the gummy dosage form in an amount from about 0.01% by weight to about 15% by weight. , gelatin is present in an amount of from about 0.5% by weight to about 8% by weight, for example from about 0.5% to about 1%, from about 1% to about 1.5%, from about 1.5% to about 2%, from about 2% to about 2.5%, from about 2.5% to about 3%, from about 3% to about 3.5%, from about 3.5% to about 4%, from about 4% to about 4.5%, from about 4.5% to about 5%, from about 5% to about 5.5%, from about 5.5% to about 6%, from about 6% to about 6.5%, from about 6.5% to about 7%, from about 7% to about 7.5%, and from about 7.5% to about 8%. Gelatin is present in an amount from about 1% by weight to about 5% by weight.

The gummy dosage form of the disclosure includes saccharides. Generally, saccharide is present in an amount from about 30% by weight to about 99% by weight of the dosage form. , sugar is present in an amount from about 40% by weight to about 95% by weight, for example, from about 40% to about 45%, from about 45% to about 50%, from about 50% to about 55%, from about 55% to about 60%, from about 60% to about 65%, from about 65% to about 70%, from about 70% to about 75%, from about 75% to about 80%, from about 80% to about 85%, from about 85% to about 90%, and from about 90% to about 85%.

In some embodiments, the gummy dosage form includes a polyol. Polyols are also referred to as sugar alcohols. Without being bound by any theory, the presence of a polyol is believed to promote the stability of the gummy dosage form of the disclosure.

Suitable polyols include, for example, hydrogenated starch hydrolysates, isomalt, lactitol, maltitol, mannitol, sorbitol, erythritol, and xylitol. Combinations of polyols may be used. , the polyol is hydrolyzed starch hydrolysates (HSH). HSH typically contains substantial quantities of hydrogenated oligo- and poly-saccharides in addition to monomeric and dimeric polyols. HSH is commonly known to include polyglycitol. An example of a commercially available HSH is Hystar.RTM. 3375 syrup (75% solids), Hystar.RTM. 4075 and Hystar.RTM. 6075 supplied by SPI Polyols. Other commercially available HSH include 75/400 from Roquette and Stabilite.RTM. liquid HSH and Stabilite.RTM. powdered HSH supplied by Corn Products Specialty Ingredients.

One or more polyols may be present in the gummy dosage form in an amount from about 30% by weight to about 99% by weight. In one embodiment, one or more polyols may be present in an amount from about 40% by weight to about 90% by weight, for example, about 40% to about 50%, about 50% to about 60%, about 60% to about 70%, about 70% to about 80%, and about 80% to about 90%. In one embodiment, one or more polyols may be present in an amount from about 40% by weight to about 60% by weight.

In embodiments in which one or more polyols are present, the ratio of polyol to sugar is typically from about 1:10 to about 10:1 by dry weight. , the ratio of polyol to sugar is from about 1:2 to about 2:1 by dry weight, for example, from about 1:1.5 to about 1:5.1.

In other embodiments, the ratio of polyol to gelling agent is from about 40:1 to about 1:1 by dry weight. , the ratio of polyol to gelling agent is from about 30:1 to about 10:1 by dry weight.

In some embodiments, the gummy dose form includes corn syrup, tapioca syrup, maple syrup, beet syrup, malt, or any other syrup. The syrup may be present without a polyol. In one embodiment, corn syrup may be present in addition to a polyol. Any suitable corn syrup may be used, for example, corn syrup having 36-65 DE (dextrose equivalents), corn syrup 42-43 DE. Corn syrup may contain about 50% by weight to about 90% by weight solids, about 80% solids.

Syrup may be present in the gummy dosage form in an amount from about 30% by weight to about 99% by weight. , corn syrup may be present in an amount from about 40% by weight to about 90% by weight, for example, about 40% to about 50%, about 50% to about 60%, about 60% to about 70%, about 70% to about 80%, and about 80% to about 90%.

In embodiments in which syrup is present, the ratio of syrup to sugar is typically from about 1:10 to about 10:1 by dry weight. In one embodiment, the ratio of the syrup to sugar is from about 1:2 to about 2:1 by dry weight, for example, from about 1:1.5 to about 1:5.1.

In other embodiments, the ratio of the syrup to gelling agent is from about 20:1 to about 1:1 by dry weight. In one embodiment, the ratio of the syrup to gelling agent is from about 10:1 to about 2:1 by dry weight.

The gummy dosage form may optionally include a pH adjusting agent. Any suitable pH adjusting agent may be used that is sufficient to adjust the pH during the manufacture of the dosage form to yield the desired pH. By way of example, the pH adjusting agent may be sodium citrate, citric acid, sodium ascorbate and ascorbic acid. Two or more pH adjusting agents may be used. The pH adjusting agent may be supplied in solid form (e.g., as a powder) or in aqueous solution. For example, citric acid may be supplied in a 50% solution. , the pH adjusting agent is sodium citrate or citric acid. In one embodiment, both sodium citrate and citric acid are included in the gummy dosage form as pH adjusting agents.

The pH adjusting agent may be present in the gummy dosage form in an amount from about 0.1% by weight to about 5% by weight. , the pH adjusting agent may be present in an amount from about 1% to about 5% by weight, for example, from about 1% to about 1.5%, from about 1.5% to about 2%, from about 2% to about 2.5%, from about 2.5% to about 3%, from about 3% to about 3.5%, from about 3.5% to about 4.0%, from about 4% to about 4.5%, and from about 4.5% to about 5%.

In some embodiments, sodium citrate is present in an amount from about 0.1% by weight to about 1% by weight. In one embodiment, sodium citrate is present in an amount from about 0.1% by weight to about 0.5% by weight, for example, from about 0.1% to about 0.2%, from about 0.2% to about 0.3%, from about 0.3% to about 0.4%, and from about 0.4% to about 0.5%.

In other embodiments, citric acid is present (as 50% aqueous solution) in an amount from about 0.5% by weight to about 3% by weight, for example from about 0.5% to about 1%, from about 1% to about 1.5%, from about 1.5% to about 2%, from about 2% to about 2.5%, and from about 2.5% to about 3%.

In certain embodiments, the gummy dosage form contains glycerin, also commonly known as glycerol. Without being bound by any theory, glycerin is believed to function as an emollient to stability the dosage form during its preparation. , glycerin USP is used. In some embodiments, glycerin is present in the gummy dosage form in addition to the absence of gelatin.

Glycerin may be present in the gummy dosage form in an amount from about 0.1% by weight to about 10% by weight. , glycerin is present in an amount from about 0.5% by weight to about 5% by weight, for example from about 0.5% to about 1%, from about 1% to about 1.5%, from about 1.5% to about 2.0%, from about 2.0% to about 2.5%, from about 2.5% to about 3.0%, from about 3.0% to about 3.5%, from about 3.5% to about 4.0%, from about 4.0% to about 4.5%, and from about 4.5% to about 5.0%.

In some embodiments, the gummy dosage form contains a flavorant. Any suitable foodgrade flavorant may be used to suppress the bitterness of the active ingredients to provide a pleasant taste to the dosage form upon chewing and swallowing. A mixture of two or more flavorants may be used to yield the desired taste characteristic.

Suitable flavorants include artificial sweeteners such as, for example, sucralose, acesulfame potassium, stevia, sodium saccharine, erythritol, and aspartame. Another suitable flavorant may be a fraction of the lactone group such as, for example, decalactone and dodecalactone (e.g., gamma dodecalactone). Lactone fractions are typically supplied in a propylene glycol solution, in particular from 0.5% to 1% in propylene glycol solution. The flavorant may be orange or cherry flavors. In one embodiment, the flavorant may be menthol.

In one embodiment, the flavorant is an artificial sweetener. In one embodiment, the artificial sweetener is sucralose.

The flavorant may be present in an amount up to about 1% by weight, up to about 0.5% by weight, for example, up to about 0.01%, up to about 0.05%, up to about 0.1%, up to about 0.2%, up to about 0.3%, up to about 0.4%, and up to about 0.5%. In certain embodiments, the amount of flavorant present is in a range bounded by any of the foregoi ng values. Fractions of the lactone group may be present in an amount of from about 1 ppm to 50 ppm, from about 2 ppm to about 10 ppm, and more from about 3 ppm to about 9 ppm.

A colorant may optionally be added to provide a suitable appearance for the gummy dosage form. Examples of suitable colorants include red or yellow dyes such as FD&C Red #40 and FD&C Yellow #6. Two or more colorants may be combined.

The gummy dosage form of the disclosure generally has a water content, also referred to as a residual moisture content, of less than about 15% by weight, e.g., about 14% or less, about 13% or less, about 12% or less, about 11% or less, about 10% or less, about 9% or less, about 8% or less, about 7% or less, about 6% or less, or about 5% or less. In other embodiments, the water content of the gummy dosage form is in a range bounded by any of the foregoing values. In one embodiment, the water content of the gummy dosage form is from about 8% by weight to about 15% by weight.

In some instances, the gummy dosage form comprises one or more active agents, a gelling agent, gelatin, sugar, a polyol, and a pH adjusting agent. In other instances, the gummy dosage form comprises one or more active agents, a gelling agent, gelatin, sugar, corn syrup, and a pH adjusting agent. In other instances, the gummy dosage form comprises one or more active agents, a gelling agent, sugar, a polyol, glycerin, and a pH adjusting agent.

In some instances, the gummy dosage form comprises one or more active agents, pectin, sugar, hydrolyzed starch hydrolysate, hydrolyzed gelatin, and a pH adjusting agent. In other instances, the gummy dosage form comprises one or more active agents, pectin, sugar, corn syrup, hydrolyzed gelatin, and a pH adjusting agent.

In some instances, the gummy dosage form comprises one or more active agents, pectin, sugar, hydrolyzed starch hydrolysate, glycerin, and a pH adjusting agent.

In some instances, the gummy dosage form comprises: one or more active agents in an amount from about 0.01% by weight to about 10% by weight; pectin in an amount from about 0.5% by weight to about 7% by weight; sugar in an amount from about 40% by weight to about 95% by weight; optionally hydrolyzed starch hydrolysate in an amount from about 40% by weight to about 90% by weight; optionally hydrolyzed gelatin in an amount from about 0.5% by weight to about 8% by weight; sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: one or more active agents in an amount from about 0.01% by weight to about 10% by weight; pectin in an amount from about 0.5% by weight to about 7% by weight; sugar in an amount from about 40% by weight to about 95% by weight; syrup in an amount from about 40% by weight to about 90% by weight; hydrolyzed gelatin in an amount from about 0.5% by weight to about 8% by weight; sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: one or more active agents in an amount from about 0.01% by weight to about 10% by weight; pectin in an amount from about 0.5% by weight to about 7% by weight; sugar in an amount from about 40% by weight to about 95% by weight; hydrolyzed starch hydrolysate in an amount from about 40% by weight to about 90% by weight; glycerin in an amount from about 0.1% by weight to about 5% by weight; sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: chlorpheniramine maleate; phenylephrine hydrochloride; pectin in an amount from about 0.2% by weight to about 6% by weight; sugar in an amount from about 40% by weight to about 95% by weight; cyclodextrin in an amount from 0% to 10% by weight, sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: diphenhydramine liydochloride; pectin in an amount from about 0.2% by weight to about 6% by weight; sugar in an amount from about 40% by weight to about 95% by weight; cyclodextrin in an amount from 0% to 10% by weight, sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: loratadine; pectin in an amount from about 0.2% by weight to about 6% by weight; sugar in an amount from about 40% by weight to about 95% by weight; cyclodextrin in an amount from 0% to 10% by weight, sodium citrate in an amount from about 0. 1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: cetirizine; pectin in an amount from about 0.2% by weight to about 6% by weight; sugar in an amount from about 40% by weight to about 95% by weight; cyclodextrin in an amount from 0% to 10% by weight, sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: aspirin; pectin in an amount from about 0.2% by weight to about 6% by weight; sugar in an amount from about 40% by weight to about 95% by weight; cyclodextrin in an amount from 0% to 10% by weight, sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: pamoate; pectin in an amount from about 0.2% by weight to about 6% by weight; sugar in an amount from about 40% by weight to about 95% by weight; cyclodextrin in an amount from 0% to 10% by weight, sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: naproxen; pectin in an amount from about 0.2% by weight to about 6% by weight; sugar in an amount from about 40% by weight to about 95% by weight; cyclodextrin in an amount from 0% to 10% by weight, sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: acetaminophen; pectin in an amount from about 0.2% by weight to about 6% by weight; sugar in an amount from about 40% by weight to about 95% by weight; cyclodextrin in an amount from 0% to 10% by weight, sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: Sildenafil Citrate, pectin in an amount from about 0.2% by weight to about 6% by weight; sugar in an amount from about 40% by weight to about 95% by weight; cyclodextrin in an amount from 0% to 10% by weight, sodium citrate in an amount from about 0.1% by weight to about 1% by weight, and citric acid in an amount from about 0.5% by weight to about 3% by weight, wherein the water content of the gummy dosage form is from about 8% by weight to about 15% by weight.

In some instances, the gummy dosage form comprises: Tada:lafl; pectin in an amount from about 0.2% by weight to about 6% by weight; sugar in an amount from about 40% by weight to about 95% by weight; cyclodextrin in an amount from 0% to 10% by weight, sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: Vardenafil hydrochloride; pectin in an amount from about 0.2% by weight to about 6% by weight; sugar in an amount from about 40% by weight to about 95% by weight; cyclodextrin in an amount from 0% to 10% by weight, sodium citrate in an amount from about 0.1% by weight to about 1% by weight; and citric acid in an amount from about 0.5% by weight to about 3% by weight, wherein the water content of the gummy dosage form is from about 8% by weight to about 1 5% by weight.

In some instances, the gummy dosage form comprises: metaformin; pectin in an amount from about 0.2% by weight to about 6% by weight; sugar in an amount from about 40% by weight to about 95% by weight; sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: cannabinoids; pectin in an amount from about 0.2% by weight to about 6% by weight; sugar in an amount from about 40% by weight to about 95% by weight; cyclodextrin in an amount from 0% to 10% by weight, sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: an active agent selected from the group consisting of an antacid, an anti-foaming agent, a histamine H2-receptor antagonist, a proton pump inhibitor, or a combination thereof in an amount from about 0.01% by weight to about 10% by weight; pectin in an amount from about 0.5% by weight to about 7% by weight; sugar in an amount from about 40% by weight to about 95% by weight; hydrolyzed starch hydrolysate in an amount from about 40% by weight to about 90% by weight; hydrolyzed gelatin in an amount from about 0.5% by weight to about 8% by weight; sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: an active agent selected from the group consisting of an antacid, an anti-foaming agent, a histamine H2-receptor antagonist, a proton pump inhibitor, or a combination thereof in an amount from about 0.01% by weight to about 10% by weight; pectin in an amount from about 0.5% by weight to about 7% by weight; sugar in an amount from about 40% by weight to about 95% by weight; corn syrup in an amount from about 40% by weight to about 90% by weight; hydrolyzed gelatin in an amount from about 0.5% by weight to about 8% by weight; sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

In some instances, the gummy dosage form comprises: calcium carbonate, simethicone, famotidine, or omeprazole; pectin in an amount from about 0.5% by weight to about 7% by weight; sugar in an amount from about 40% by weight to about 95% by weight; hydrolyzed starch hydrolysate in an amount from about 40% by weight to about 90% by weight; hydrolyzed gelatin in an amount from about 0.5% by weight to about 8% by weight; sodium citrate in an amount from about 0.1% by weight to about 1% by weight; and citric acid in an amount from about 0.5% by weight to about 3% by weight, wherein the water content of the gummy dosage form is from about 8% by weight to about 15% by weight.

The gummy dosage form of the disclosure can be prepared by any suitable method including, for example, a batch process or a continuous process. In some embodiments, the components of the dosage form are first combined together in a suitable vessel. The components can be combined in any suitable order.

In some embodiments, different blends of components are prepared separately and then combined together to form a final blend from which the gummy dosage form is obtained. For example, a primary blend may be combined with a secondary blend to form the final blend. A separate blend containing flavorants and/or colorants and an acid solution may optionally be added in the preparation of the final blend.

In one instance, a primary blend is prepared by combining pectin, sugar, a polyol, and a pH adjusting agent with water. In one instance, the primary blend may be prepared by combining pectin, sugar, corn syrup, and a pH adjusting agent with water. A pH adjusting agent such as, for example, sodium citrate may optionally be added to the primary blend. In some embodiments, the primary blend has a pH from about 2 to about 6, from about 2.5 to about 4, and more from about 2.8 to about 3.8.

In certain aspects, the primary blend is cooked at an appropriate temperature and for an appropriate length of time to provide the primary blend with any suitable moisture content for further processing. In one embodiment, the primary blend has a moisture content after cooking from about 5% by weight to about 25% by weight. In one embodiment, the primary blend has a residual moisture content after cooking from about 9% by weight to about 20% by weight, for example, about 9% to about 10%, about 10% to about 11%, about 11% to about 12%, about 12% to about 13%, about 13% to about 14%, and about 14% to about 15%, about 15% to about 16%, about 16% to about 17%, about 17% to about 18%, about 18% to about 19%, and about 19% to about 20%. Generally, the primary blend may be cooked at a temperature of about 230 F (110°C) to about 250 F (121.1°C), for example, about 230 F (110°C) to about 235 F (112.8°C), about 235 F (112.8°C) to about 240 F (115.6°C), about 240 F (115.6°C) to about 245 F (118.3°C), and about 245 F (118.3°C) to about 250 F (121.1°C)

A secondary blend may be added to the primary blend after cooking is completed. The secondary blend may contain one or more components of the gummy dosage form. In some embodiments, the secondary blend includes chlorpheniramine maleate, phenylephrine hydrochloride, and hydrolyzed gelatin. In other embodiments, the secondary blend includes calcium carbonate, simethicone, famotidine, or omeprazole, and hydrolyzed gelatin. Water may be added to the secondary blend to dissolve water-soluble components and/or form a homogenous mixture. Other components may be added to the secondary blend including, for example, glycerin, flavorants and colorants. In one embodiment, an additional blend may be prepared containing glycerin, flavorants and colorants. An acid solution may further be prepared containing citric acid to obtain the desired pH of the final blend. The final blend may be obtained by combining the primary blend, secondary blend, additional blend and citric acid in any order.

The final blend may be further processed as needed prior to preparation of the gummy dosage form. For example, the final blend may be transferred to a depositor hopper having a jacket to maintain a temperature of from about 180 F (82.2°C) to about 210 F (98.9°C), about 190 (87.8°C) to about 200 F (93.3°C). After a suitable amount of time, the final blend may be dispensed from the depositor hopper to product the gummy dosage form of the disclosure.

The gummy dosage form may be obtained by depositing the final blend into pre-formed plastic molds using conventional techniques. In one embodiment, the plastic molds are blister packs having multiple cavities that provide for unit dose packaging of the gummy dosage form without having to transfer the dosage form from a mold to a separate container. The dosage form solidifies in the plastic molds, which serve as the final packaging. As the temperature of the dosage form cools, the dosage form takes its final shape in the cavities of the blister pack. The blister pack is sealed, for example, using foil. One or more blister packs may be packaged in containers. In one embodiment, the dosage forms may be prepared in molds and transferred to other suitable containers.

In one embodiment, a pre-determined amount of the final blend, for example based on weight, is dispensed into each cavity to form individual pieces. The individual pieces contain the desired amount of the active ingredients as described herein. For example, individual pieces may contain 4 mg chlorpheniramine maleate and 10 mg phenylephrine hydrochloride for an adult dose and 2 mg chlorpheniramine maleate and 5 mg phenylephrine hydrochloride for a pediatric dose.

The following examples further illustrate the disclosure but, of course, should not be construed as in any way limiting its scope.

### EXAMPLES

### Example 1. Pectin Aspirin

Mix 7.1 g Na Citrate 8.4 g K Citrate, 145.1 g Pectin, and 310.0 g Sucrose until homogeneous to provide a gelling solution. 245 g of water was heated to 200 F (93.3°C). The above mix was added to the hot water with mixing. The solution was brought to the boiling point and boiled for 5 minutes with stirring. 409 g of boiling glucose syrup was slowly added with stirring. Brix of the solution was 73.5. The entire mix was heated until Brix 83.

In a separate container was mixed 10.8 g of aspim, 0.65 g Caffeine, and 2.2 g calcium hydroxide. To this was added 20.0 g water and mixed. This mix was then added to the Brix 83 pectin/sugar solution with stirring.

In a separate container was mixed 7.2 g 50% citric acid solution and 12.0 g pure lime extract. This solution was added to the total mixture with stirring. 0.4 g of green food color was added. Brix was 81.5

The batter was added to silicone trays. The gummy set up within 15 minutes.

### Example 2. Diphenhydramine Hydrochloride Containing Pectin Gummy

Ingredients include: 5.500 grams Sodium Bicarbonate, 3.673 grams Potassium Hydroxide food grade, 1.555 grams Diphenhydramine Hydrochloride, 145.3 grams Citrus Pectin, 500 grams Water, 409.1 grams Glucose Syrup, 310.3 grams Sucrose, 15.0 grams 50% citric acid in water, 4.5 grams Cady Apple Green, and 0.6 grams Green Food Color.

Combine 500 mL water, potassium hydroxide and sodium bicarbonate, allow the bases to dissolve. Add the pectin. Stir until homogenous and let stand for 15 minutes. Bring to a boil for one minute. The glucose syrup was then added after the minute of boiling. The solution was brought back to a boil. The sucrose was then added and the system heated until Brix 84.

The solution was cooled to 210 °F (98.9°C) and a mixture of citric acid solution, candy apple green flavor and diphenhydramine hydrochloride was added. These were mix into the batter until homogenous. Green food color was then added.

The solution was then added to silicone molds and allowed to cool to room temperature. Excellent tasting gummies resulted. Each 7. gram gummy had roughly 13 mg diphenhydramine hydrochloride.

### Example 3. Multivitamin and herbal extract gummy

Ingredient list: 21.6g Collagen, 310.0 g Sucrose, 2.3 g Angelica Sinensis Extract, 145.2 g Candy Pectin by Pacific Pectin, 4.0 g Citric Acid. The ingredients were mixed together until homogeneous.

500 mL was added to a container and heated until 200 °F *(93.3°C).* The above mix was slowly added to the water with stirring and mixed with the water until homogeneous. The mix was brought to the brink of boiling and 0.75 g of coconut oil was added with stirring. The solution was brought to a boil and allowed to boil with stirring for 5 minutes at which time 409.1 g of boiling glucose syrup was added. Brix at this point was 73. Cook until Brix 81 and add 15.2 g vitamin premix and stir until homogenous. The heat was reduced but residual heat raised the brix level to 86-87.

15.2 g 50% Citric Acid Solution and 12.1 g Strawberry Extract were combined and added to the gummy batter. 0.75 g of red food color was then added to the gummy batter. The brix level was 85.

The gummy batter was then added to silicone molds. While the gummies did set up, they could not support their own weight and would flow.

### Example 4. Multivitamin and herbal extract gummy

Ingredient list: 22.0g Collagen, 310.0 g Sucrose, 2.3 g Angelica Sinensis Extract, 146.3 g Candy Pectin by Pacific Pectin, 4.0 g Citric Acid. The ingredients were mixed together until homogeneous.

500 mL was added to a container and heated until 200 °F (93.3°C). The above mix was slowly added to the water with stirring and mixed with the water until homogeneous. The mix was brought to the brink of boiling and 0.75 g of coconut oil was added with stirring. The solution was brought to a boil and allowed to boil with stirring for 5 minutes at which time 409.1 g of boiling glucose syrup was added. Cook until Brix 80 and add 15.3 g vitamin premix (multivitamins with minerals) and stir until homogenous. The heat was reduced but residual heat raised the brix level to 89.

15.0 g 50% Citric Acid Solution and 12.0 g Strawberry Extract were combined and added to the gummy batter. 0.75 g of red food color was then added to the gummy batter. The brix level was 87 at the time of addition to molds.

The gummy batter was then added to silicone molds. While the gummies did set up and were very chewy. They would support their own weight and would hold their shape.

### Example 5. Multivitamin and herbal extract gummy

Ingredient list: 22.3g Collagen, 310.0 g Sucrose, 2.3 g Angelica Sinensis Extract, 145.2 g Candy Pectin by Pacific Pectin. The ingredients were mixed together until homogeneous. 250mL was added to a container and heated until 200 °F (93.3°C) and reduced to 205 g. The above mix was slowly added to the water with stirring and mixed with the water until homogeneous. The mix was brought to the brink of boiling and 0.75 g of coconut oil was added with stirring. The solution was brought to a boil and allowed to boil with stirring for 5 minutes at which time 407.6 g of boiling glucose syrup was added. Brix at this point was 77. Cook until Brix 80 and add 15.3 g vitamin premix and stir until homogenous. Heat until Brix 85 and add 4.0 g citric acid.

15.1 g 50% Citric Acid Solution and 12.1 g Strawberry Extract were combined and added to the gummy batter. 0.65 g of red food color was then added to the gummy batter. The brix level was 86 at the point of addition to molds.

The gummy batter was then added to silicone molds. While the gummies did set up and were chewy but not as chewy as when they were at Brix 87. They would support their own weight and would hold their shape.

### Example 6. Aspirin gummy

Ingredient list: 285.0 grams Water, 40.3 grams pectin; 100.0 grams Sucrose; 3.0 grams Sodium Citrate; 220.0 grams Sucrose; 6.0 grams Coconut Oil; 468.0 grams Boling Corn Syrup; 4.0 grams Pure Lime Extract; 11.0 grams Calcium Acetylsalicylate; 22.0 grams Maltodextrin; 20.0 grams 50% Citric Acid solution (in 50% glycerol/water); 5.0 grams glycerol; 1.4 grams Green color

Method: In a separate container is added the pectin, 100 grams of sucrose, and the sodium citrate. The components are mixed until homogeneous. This is mix 1. In a separate container calcium acetyl salicylate and maltodextrin are combined and shifted together until homogeneous. This is Mix 2. In a separate container is added the citric acid solution, green color, and lime flavor. All is mixed and warmed to 175 °F (79.4°C) until all is dissolved. This is Mix 3.

Water is heated to 200 °F (93.3°C) in a saucepan. To the hot water is added Mix 1 with stirring. The mixture is stirred until the pectin fully swells and disperses which takes roughly 3-5 minutes. The solution is brought to a boil. To the boiling Mix 1 solution is added the remaining sucrose with stirring. The sucrose is allowed to dissolve.

Coconut oil is then added dropwise to the boiling mixture with stirring.

The boiling corn syrup is then added to the boiling pectin/sugars/oil mix. And the system heated until Brix 75. Mix 2 was then slowly sprinkled into the Brix 75 solution to avoid clumping. The mixture was heated to Brix 82 at which time Mix 3 was added dropwise with stirring until completely homogenous.

The gummy batter was then added to silicone molds.

### Example 7. Aspirin, N-Acetylglucosamine gummy

Ingredient List: 375.0 grams Water; 5.0 grams Glycerol; 10.8 grams Acetylsalicylic Acid; 0.7 grams Caffeine; 2.2 grams Calcium Hydroxide; 40.0 grams pectin; 50.0 grams N-acetyl glucosamine; 50.0 grams Fructose; 3.0 grams Sodium Citrate; 20.0 grams g-cyclodextrin; 80.0 grams Fructose; 120.0 grams Sucrose; 410.0 grams Boling Glucose Syrup; 2.0 grams Lime Flavor Water Soluble; 2.0 grams Lime Flavor Oil Soluble; 25.0 grams 50% Citric Acid solution (in 50% glycerol/water); 1.2 grams Green color

Method: In a separate container is added the pectin, 50 grams of fructose, 50 grams N-acetyl glucosamine and the sodium citrate. The components are mixed until homogeneous. This is mix 1. In a separate container is added cyclodextrin, fructose, and sucrose and these are fully mixed. This is mix 2. In a separate container is added the citric acid solution, green color, and lime flavor. All is mixed and warmed to 175 °F (79.4°C) until all is dissolved. This is Mix 3.

Water is heated to 200 °F (93.3°C) in a saucepan. To the hot water is added calcium hydroxide and caffeine and both are allowed to fully dissolve. To the hot water is then added the aspirin and the aspirin is allowed to fully dissolve. Glycerol is then added.

To the hot water is added Mix 1 with stirring. The mixture is stirred until the pectin fully swells and disperses which takes roughly 3-5 minutes. The solution is brought to a boil.

To the boiling Mix 1 solution is added Mix 2 with stirring. The Mix 2 is allowed to dissolve.

The boiling corn syrup is then added to the boiling pectin/sugars/oil mix. And the system heated to Brix 80 at which time the system was cooled to 220 (104.4°C) - 230 °F (110°C) and Mix 3 was added dropwise with stirring until completely homogenous.

The gummy batter was then added to silicone molds.

### Example 8. CBD gummy

Ingredient List: 400 grams Water; 37.5 grams Pectin; 220.0 grams Sucrose; 50.0 grams Fructose; 3.0 grams Sodium Citrate; 5.0 grams Glycerol; 20.0 grams Mannitol; 20.0 grams g-Cyclodextrin; 7.5 grams Coconut Oil; 410.0 grams Glucose Syrup; 2.2 grams RSO CBD Serum 20: 1 (63.0% CBD); 75 grams 50 % Citric Acid solution (50-50 water glycerol); 5.1 grams Cochineal Pink; 4.0 grams Watermelon Flavor

Method: To a container was added the water which is then heated to 200 °F (93.3°C). Glycerol was then added to the water. To a separate container was added the pectin, 100 grams of sucrose and the sodium citrate and mixed until homogeneous. This mixture was then added to the hot water with rapid mixing. The water becomes more viscous as the pectin dissolved. The water was brought to a gentle boil and the mixing continued for 3-5 minutes.

To a separate container are added the remainder of the sucrose, fructose, mannitol and cyclodextrin. These components are mixed until homogenous. The sugar mixture was then added to swelled pectin solution above with rapid mixing and maintain the boil.

To a separate container was added the glucose syrup. The glucose syrup was brought to a boil and reduced to a Brix 85. The boiling glucose syrup was then slowly added to the pectin solution slowly with rapid mixing. Coconut oil and RSO CBD Serum 20:1 were added with mixing. Brix was at 70. The mixture was brought to a strong boil.

The system was then heated to Brix 79. The system was cooled to 210 (98.9°C)- 220 °F (104.4°C) at which time the color was added followed by the flavors and then the citric acid solution. The completed gummy batter was then poured into molds.

### Example 9. Cetirizine gummy

Ingredient List: 285.0 grams Water; 40.3 grams pectin; 101.0 grams Sucrose; 3.0 grams Sodium Citrate; 235.0 grams Sucrose; 14.0 grams Clusterdextrin; 20.0 grams Mannitol; 6.0 grams Coconut Oil; 410.0 grams Boling Glucose Syrup; 6.5 grams Blood Orange Extract; 1.30 grams Cetirizine Dihydrochloride; 20.0 grams 50% Citric Acid solution (in 50% glycerol/water); 5.0 grams glycerol;0.6 grams orange color

Method: In a separate container is added the pectin, 100 grams of sucrose, and the sodium citrate. The components are mixed until homogeneous. This is mix 1. In a separate container is added the remaining sucrose, cluster dextrin, and mannitol. The components are mixed until homogeneous. This is mix 2.

In a separate container is added the citric acid solution, orange color, blood orange flavor and the cetirizine. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil. To the boiling Mix 1 solution is added Mix 2 with stirring.

Coconut oil is then added dropwise to the boiling mixture with stirring. The boiling glucose syrup is then added to the boiling pectin/sugars/oil mix. The mixture was heated to Brix 80 at which time Mix 3 was added dropwise with stirring. The gummy batter was then added to silicone molds.

Example 10. Cetirizine Gummy that is safer for diabetics and lowered instance of tooth decay.

Ingredient List: 285.0 grams Water; 40.3 grams pectin; 100.0 grams Trehalose; 3.0 grams Sodium Citrate; 167.5 grams Palatinose; 67.5 grams Trehalose; 15.0 grams b-cylcodextrin; 20.0 grams Mannitol; 6.0 grams Coconut Oil; 410.0 grams Boling Psicose Syrup; 6.5 grams Blood Orange Extract; 1.30 grams Cetirizine Dihydrochloride; 20.0 grams 50% Citric Acid solution (in 50% glycerol/water); 5.0 grams glycerol; 0.6 grams orange color

Method: In a separate container is added the pectin, 100 grams of trehalose, and the sodium citrate. The components are mixed until homogeneous. This is mix 1.
In a separate container is added the remaining trehalose, palatinose, cyclodextrin, and mannitol. The components are mixed until homogeneous. This is mix 2. In a separate container is added the citric acid solution, orange color, blood orange flavor and the cetirizine. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil.

Mix 2 is added to the boiling psicose syrup which is then added to the boiling pectin/sugars/oil mix. Coconut oil is then added dropwise to the boiling mixture with stirring. The mixture was heated to Brix 79 at which time Mix 3 was added dropwise with stirring. The gummy batter was then added to silicone molds.

### Example 11. Low Glycemic Index Cetirizine Gummy

Ingredient List: 285.0 grams Water; 40.3 grams pectin; 100.0 grams Trehalose; 3.0 grams Sodium Citrate; 167.5 grams Palatinose; 67.5 grams Trehalose; 15.0 grams b-cylcodextrin; 20.0 grams Mannitol; 410.0 grams Boling Psicose Syrup; 6.5 grams Orange Flavor USP; 1.32 grams Cetirizine Dihydrochloride; 20.0 grams 50% Citric Acid solution (in 50% glycerol/water); 5.0 grams glycerol; 0.6 grams orange color

Method: In a separate container is added the pectin, 100 grams of trehalose, cetirizine dihydrochloride, cyclodextrin and the sodium citrate. The components are mixed until homogeneous. This is mix 1. In a separate container is added the remaining trehalose, palatinose, and mannitol. The components are mixed until homogeneous. This is mix 2. In a separate container is added the citric acid solution, orange color, and orange flavor. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil.

Mix 2 is added to the boiling psicose syrup which is then added to the boiling pectin/sugars/oil mix. The mixture was heated to Brix 79 at which time Mix 3 was added dropwise with stirring. The gummy batter was then added to silicone molds.

### Example 12. Cetirizine Gummy

Ingredient List: 285.0 grams Water; 40.3 grams pectin; 100.0 grams Sucrose; 3.0 grams Sodium Citrate; 130 Grams Sucrose; 100 grams Fructose; 15.0 grams b-cyclodextrin; 20.0 grams Mannitol; 410.0 grams Boling Glucose Syrup; 6.5 grams Orange Flavor USP; 1.32 grams Cetirizine Dihydrochloride; 20.0 grams 50% Citric Acid solution (in 50% glycerol/water); 5.0 grams glycerol; 0.6 grams orange color

Method: In a separate container is added the pectin, 100 grams of sucrose, cetirizine dihydrochloride, cyclodextrin and the sodium citrate. The components are mixed until homogeneous. This is mix 1.In a separate container is added the remaining sucrose, fructose, and mannitol. The components are mixed until homogeneous. This is mix 2. In a separate container is added the citric acid solution, orange color, and orange flavor. All is mixed and warmed to 175 ° F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/sugars/oil mix. The mixture was heated to Brix 82 at which time Mix 3 was added dropwise with stirring. The gummy batter was then added to silicone molds.

### Example 13. Loratadine Gummy

Ingredient List: 285.0 grams Water; 40.3 grams pectin; 100.0 grams Sucrose; 3.0 grams Sodium Citrate; 130 Grams Sucrose; 100 grams Fructose; 15.0 grams b-cyclodextrin; 20.0 grams Mannitol; 410.0 grams Boling Glucose Syrup; 6.5 grams Orange Flavor USP; 1.42 grams Loratadine Hydrochloride; 20.0 grams 50% Citric Acid solution (in 50% glycerol/water); 5.0 grams glycerol; 0.6 grams orange color

Method: In a separate container is added the pectin, 100 grams of sucrose, loratadine hydrochloride, cyclodextrin and the sodium citrate. The components are mixed until homogeneous. This is mix 1. In a separate container is added the remaining sucrose, fructose, and mannitol. The components are mixed until homogeneous. This is mix 2. In a separate container is added the citric acid solution, orange color, and orange flavor. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/sugars/oil mix. The mixture was heated to Brix 79 at which time Mix 3 was added dropwise with stirring. The gummy batter was then added to silicone molds.

### Example 14. Tadalafil Gummy

Ingredient List: 285.0 grams Water; 40.3 grams pectin; 100.0 grams Sucrose; 3.0 grams Sodium Citrate; 130 Grams Sucrose; 100 grams Fructose; 15.0 grams b-cyclodextrin; 20.0 grams Mannitol; 410.0 grams Boling Glucose Syrup; 6.5 grams Orange Flavor USP; 1.36 grams Tadalafil Hydrochloride; 20.0 grams 50% Citric Acid solution (in 50% glycerol/water); 5.0 grams glycerol; 4.5 grams Root Beer flavor; 0.6 grams brown caramel color

Method: In a separate container is added the pectin, 100 grams of sucrose, tadalafil hydrochloride, cyclodextrin and the sodium citrate. The components are mixed until homogeneous. This is mix 1.

In a separate container is added the remaining sucrose, fructose, and mannitol. The components are mixed until homogeneous. This is mix 2. In a separate container is added the citric acid solution, brown color, and root beer flavor. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/sugars/oil mix. The mixture was heated to Brix 79 at which time Mix 3 was added dropwise with stirring. The gummy batter was then added to silicone molds.

### Example 14. Tadalafil Gummy

Ingredient List: 285.0 grams Water; 40.3 grams pectin; 100.0 grams Sucrose; 3.0 grams Sodium Citrate; 130 Grams Sucrose; 100 grams Fructose; 15.0 grams b-cyclodextrin; 20.0 grams Mannitol; 410.0 grams Boling Glucose Syrup; 2.80 grams Tadalafil Hydrochloride; 20.0 grams 50% Citric Acid solution (in 50% glycerol/water); 5.0 grams glycerol; 4.5 grams Bourbon Flavor; 0.6 grams brown caramel color

Method: In a separate container is added the pectin, 100 grams of sucrose, ½ cyclodextrin and the sodium citrate. The components are mixed until homogeneous. This is mix 1.

In a separate container is added the remaining sucrose, ¼ cyclodextrin, fructose, and mannitol. The components are mixed until homogeneous. This is mix 2. In a separate container is added the citric acid solution, ¼ cyclodextrin, tadalafil hydrochloride, brown color, and bourbon flavor. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/sugars/oil mix. The mixture was heated to Brix 79 at which time Mix 3 was added dropwise with stirring. The gummy batter was then added to silicone molds.

### Example 15. Vardenafil Gummy

Ingredient List: 285.0 grams Water; 40.3 grams pectin; 100.0 grams Sucrose; 3.0 grams Sodium Citrate; 130 Grams Sucrose; 100 grams Fructose; 15.0 grams b-cyclodextrin; 20.0 grams Mannitol; 410.0 grams Boling Glucose Syrup; 1.51 grams vardenafil hydrochloride; 20.0 grams 50% Citric Acid solution (in 50% glycerol/water); 5.0 grams glycerol; 4.5 grams Root Beer flavor; 0.6 grams brown caramel color

Method: In a separate container is added the pectin, 100 grams of sucrose, vardenafil hydrochloride, cyclodextrin and the sodium citrate. The components are mixed until homogeneous. This is mix 1.

In a separate container is added the remaining sucrose, fructose, and mannitol. The components are mixed until homogeneous. This is mix 2. In a separate container is added the citric acid solution, brown color, and root beer flavor. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/sugars/oil mix. The mixture was heated to Brix 79 at which time Mix 3 was added dropwise with stirring. The gummy batter was then added to silicone molds.

### Example 16. Sildenafil Gummy

Ingredient List: 285.0 grams Water; 40.3 grams pectin; 100.0 grams Sucrose; 3.0 grams Sodium Citrate; 130 Grams Sucrose; 100 grams Fructose; 15.0 grams b-cyclodextrin; 20.0 grams Mannitol; 410.0 grams Boling Glucose Syrup; 7.65 grams sildenafil citrate; 20.0 grams 50% Citric Acid solution (in 50% glycerol/water); 5.0 grams glycerol; 4.5 grams Raspberry flavor; 0.6 grams Red color

Method: In a separate container is added the pectin, 100 grams of sucrose, sildenafil citrate, cyclodextrin and the sodium citrate. The components are mixed until homogeneous. This is mix 1. In a separate container is added the remaining sucrose, fructose, and mannitol. The components are mixed until homogeneous. This is mix 2. In a separate container is added the citric acid solution, red color, and raspberry flavor. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/sugars/oil mix. The mixture was heated to Brix 79 at which time Mix 3 was added dropwise with stirring. The gummy batter was then added to silicone molds.

### Example 17. Sildenafil Chocolate Gummy

Ingredient List: 285.0 grams Water; 40.3 grams pectin; 100.0 grams Sucrose; 3.0 grams Sodium Citrate; 130 Grams Sucrose; 100 grams Fructose; 30 grams Cacao Powder, 15.0 grams g-cyclodextrin; 20.0 grams Mannitol; 410.0 grams Boling Glucose Syrup; 8.10 grams sildenafil citrate; 20.0 grams 50% Citric Acid solution (in 50% glycerol/water); 5.0 grams glycerol; 1.2 grams Raspberry flavor

Method: In a separate container is added the pectin, 100 grams of sucrose, sildenafil citrate, cyclodextrin and the sodium citrate. The components are mixed until homogeneous. This is mix 1. In a separate container is added the remaining sucrose, cacao powder, fructose, and mannitol. The components are mixed until homogeneous. This is mix 2. In a separate container is added the citric acid solution, and raspberry flavor. All is mixed and warmed to 175 °F (79.4°C) until all is dissolved. This is Mix 3. Water is heated to 200 °F (93.3°C) in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/sugars/oil mix. The mixture was heated to Brix 79 at which time Mix 3 was added dropwise with stirring. The gummy batter was then added to silicone molds.

### Example 18. Naproxen Gummy

Ingredient List: 285.0 grams Water; 40.3 grams pectin; 100.0 grams Sucrose; 6.0 grams Naproxen Sodium; 130 Grams Sucrose; 100 grams Fructose; 18.0 grams b-cyclodextrin; 20.0 grams Mannitol; 410.0 grams Boling Glucose Syrup; 4.5 grams Lemon Flavor USP; 20.0 grams 50% Citric Acid solution (in 50% glycerol/water); 5.0 grams glycerol; 0.8 grams Yellow Color

Method: In a separate container is added the pectin, 100 grams of sucrose, naproxen sodium, and cyclodextrin. The components are mixed until homogeneous. This is mix 1. In a separate container is added the remaining sucrose, fructose, and mannitol. The components are mixed until homogeneous. This is mix 2. In a separate container is added the citric acid solution, yellow color, and lemon flavor. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes.

The solution is brought to a boil. Mix 2 is added to the boiling glucose syrup is then added to the boiling pectin/sugars/oil mix. The mixture was heated to Brix 78 at which time Mix 3 was added dropwise with stirring. The gummy batter was then added to silicone molds.

### Example 19. Ibuprofen Gummy

Ingredient List: 285.0 grams Water; 40.3 grams pectin; 100.0 grams Sucrose; 9.0 grams Ibuprofen Lysate; 130 Grams Sucrose; 100 grams Fructose; 20.0 grams b-cyclodextrin; 20.0 grams Mannitol; 410.0 grams Boling Glucose Syrup; 4.5 grams Lime Flavor USP; 20.0 grams 50% Citric Acid solution (in 50% glycerol/water); 5.0 grams glycerol; 0.6 grams Green color

Method: In a separate container is added the pectin, 100 grams of sucrose, ibuprofen lysate, and cyclodextrin. The components are mixed until homogeneous. This is mix 1. In a separate container is added the remaining sucrose, fructose, and mannitol. The components are mixed until homogeneous. This is mix 2. In a separate container is added the citric acid solution, green color, and lime flavor. All is mixed and warmed to 175 °F (79.4°C) until all is dissolved. This is Mix 3.

Water is heated to 200 °F (93.3°C)in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/ sugars/oil mix. The mixture was heated to Brix 78 at which time Mix 3 was added dropwise with stirring. The gummy batter was then added to silicone molds.

### Example 20. Acetaminophen Gummy

Ingredient List: 285.0 grams Water; 40.3 grams pectin; 100.0 grams Sucrose; 10.1 grams acetaminophen; 130 Grams Sucrose; 100 grams Fructose; 25.0 grams b-and g-cyclodextrin (1:1); 3.0 grams Sodium Citrate, 20.0 grams Mannitol; 410.0 grams Boling Glucose Syrup; 4.5 grams Watermelon Flavor; 20.0 grams 50% Citric Acid solution (in 50% glycerol/water); 5.0 grams glycerol; 0.7 grams Red color

Method: In a separate container is added the pectin, 100 grams of sucrose, acetaminophen, cyclodextrin and the sodium citrate. The components are mixed until homogeneous. This is mix 1. In a separate container is added the remaining sucrose, fructose, and mannitol. The components are mixed until homogeneous. This is mix 2. In a separate container is added the citric acid solution, red color, and watermelon flavor. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/sugars/oil mix. The mixture was heated to Brix 78 at which time Mix 3 was added dropwise with stirring. The gummy batter was then added to silicone molds.

### Example 21. Acetaminophen Gummy

Ingredient List: 285.0 grams Water; 40.3 grams pectin; 100.0 grams Sucrose; 10.1 grams acetaminophen; 130 Grams Sucrose; 100 grams Fructose; 25.0 grams b-and g-cyclodextrin (1:1); 3.0 grams sodium citrate, 20.0 grams Mannitol; 410.0 grams Boling Glucose Syrup; 4.5 grams Watermelon Flavor; 20.0 grams 50% Citric Acid solution (in 50% glycerol/water); 5.0 grams glycerol; 0.7 grams Red color

Method: In a separate container is added the pectin, 100 grams of sucrose, ½ cyclodextrin and the sodium citrate. The components are mixed until homogeneous. This is mix 1. In a separate container is added the remaining sucrose, ¼ cyclodextrin, fructose, and mannitol. The components are mixed until homogeneous. This is mix 2. In a separate container is added the citric acid solution, ¼ cyclodextrin, acetaminophen, red color, and watermelon flavor. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/sugars/oil mix. The mixture was heated to Brix 78 at which time Mix 3 was added dropwise with stirring. The gummy batter was then added to silicone molds.

### Example 22. Metaformin Gummy

Ingredient List: 285.0 grams Water; 40.3 grams pectin; 100.0 grams Sucrose; 10.1 grams metaformin; 130 Grams Sucrose; 100 grams Fructose; 20.0 grams Mannitol; 410.0 grams Boling Glucose Syrup; 4.5 grams Peach Flavor; 25.0 grams 50% Citric Acid solution (in 50% glycerol/water); 5.0 grams glycerol; 0.3 grams Orange color

Method: In a separate container is added the pectin, 100 grams of sucrose, metaformin. The components are mixed until homogeneous. This is mix 1. In a separate container is added the remaining sucrose, fructose, and mannitol. The components are mixed until homogeneous. This is mix 2. In a separate container is added the citric acid solution, orange color, and peach flavor. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/sugars/oil mix. The mixture was heated to Brix 78 at which time Mix 3 was added dropwise with stirring. The gummy batter was then added to silicone molds.

### Example 18. Chlorpheniramine Maleate Gummy

Ingredient List: 285.0 grams Water; 40.3 grams pectin; 100.0 grams Sucrose; 2.0 grams chlorpheniramine maleate; 130 Grams Sucrose; 100 grams Fructose; 15.0 grams b-cyclodextrin; 20.0 grams Mannitol; 410.0 grams Boling Glucose Syrup; 4.5 grams Lemon Flavor USP; 20.0 grams 50% Citric Acid solution (in 50% glycerol/water); 5.0 grams glycerol; 0.8 grams Yellow Color

Method: In a separate container is added the pectin, 100 grams of sucrose, chlorpheniramine maleate, and cyclodextrin. The components are mixed until homogeneous. This is mix 1. In a separate container is added the remaining sucrose, fructose, and mannitol. The components are mixed until homogeneous. This is mix 2. In a separate container is added the citric acid solution, yellow color, and lemon flavor. All is mixed and warmed to 175 °F (79.4°C) until all is dissolved. This is Mix 3.

Water is heated to 200 °F (93.3°C) in a saucepan. To the hot water is added Mix 1. The mixture is stirred until the pectin fully swells and disperses which takes roughly 3-5 minutes. The solution is brought to a boil. Mix 2 is added to the boiling glucose syrup is then added to the boiling pectin/sugars/oil mix. The mixture was heated to Brix 78 at which time Mix 3 was added dropwise with stirring. The gummy batter was then added to silicone molds.

The use of the terms "a" and "an" and "the" and "at least one" and similar referents in the context of describing the disclosure (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted y context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the disclosure.

No language in the specification should be construed as indicating any non-claimed element as essential to the practice of the disclosure.

Embodiments of this disclosure are described herein, including the best mode known to the inventors for carrying out the disclosure. Variations of those embodiments may become apparent to those of ordinary skill in the art upon reading the foregoing description. The inventors expect skilled artisans to employ such variations as appropriate, and the inventors intend for the disclosure to be practiced otherwise than as specifically described herein.

Moreover, any combination of the above-described elements in all possible variations thereof is encompassed by the disclosure unless otherwise indicated herein or otherwise clearly contradicted by context.

## Claims

1. A method of making a gummy composition, comprising
providing a gelling blend, comprising
pectin having a carboxyl content of not more than 45% by weight of pectin and an amide content of not less than 20% by weight of pectin;
a solubilizing blend, wherein the solubilizing blend comprises a solubilizing salt having a formula of M-A, wherein M represents a cation of an alkaline or alkaline earth metal and A represents an anion of a multivalent acid having at least one pKa exceeding 4;
a swelling solvent; and
a first bonding blend,
optionally providing an emulsifying blend;
providing a second bonding blend, so that the first bonding blend and the second bonding blend form a bonding blend, wherein the bonding blend comprises a combination of at least two of a monosaccharide, a disaccharide and a tri-saccharide;
providing an active blend, comprising an active agent;
combining the gelling blend, the emulsifying blend, the bonding blend and the active blend to provide a pre-molding blend;
combining the pre-molding blend with a pH adjusting blend to provide a molding blend, wherein the molding blend has a pH value of at least 5; and
forming the molding blend into a gummy dosage composition.

2. The method of Claim 1, wherein the gelling blend comprises pectin having a carboxyl content of less than 40% by weight of pectin.

3. The method of Claim 1, wherein the gelling blend comprises pectin having an amide content from 30% to 35% by weight of pectin.

4. The method of Claim 1, wherein the gelling blend comprises pectin having a carboxyl content and an amide content, wherein the total of the carboxyl and the amide content is not from 56% to 70% by weight of pectin.

5. The method of Claim 1, wherein the gelling blend comprises pectin having a carboxyl content not more than 39% and an amide content not less than 27% by weight of pectin.

6. The method of Claim 1, wherein the swelling solvent comprises water, or a combination of water and glycerol.

7. The method of Claim 1, wherein the bonding blend comprises a combination of at least two of a monosaccharide, a disaccharide and a tri-saccharide, and wherein the bonding blend comprises tagatose, psicose, trehalose, isomaltulose, or a combination thereof.

8. The method of Claim 1, wherein the solubilizing salt and the pectin are present in such a stoichiometric weight to weight ratio such that 1/3 of the carboxylic groups in pectin are configured to be deprotonated.

9. A gummy composition, comprising a gelling blend, optionally an emulsifying blend, a bonding blend and an active agent, wherein the gelling blend comprises pectin having a carboxyl content of not more than 45% by weight of pectin and an amide content of not less than 20% by weight of pectin, wherein the gelling blend further comprises a solubilizing blend, wherein the solubilizing blend comprises a solubilizing salt having a formula M-A, wherein M represents a cation of an alkaline or alkaline earth metal and A represents an anion of a multivalent acid having at least one pKa exceeding 4, and wherein the bonding blend comprises a combination of at least two of a monosaccharide, a disaccharide and a tri-saccharide.

10. The gummy composition of Claim 9, wherein the gelling blend further comprises gelatin, wherein the collagen has a MW from 10,000 to 50,000 Dalton.

11. The gummy composition of Claim 9, wherein the active agent is selected from the following list:
a penicillin, a cephalosporin, a macrolide, a fluroquinolone, a sulfonamide, a tetracycline, an aminoglycoside, or a combination thereof; or
acetaminophen, aspirin, salicylic acid, ibuprofen, naproxen, diphenhydramine, scopolamine, metaformin, a PDE5 inhibitor, cetirizine, or a combination thereof; or
cetirizine, diphenhydramine, loratadine, chlorpheniramine, brompheniramine, alimemazine, cyprohetadine, doxylamine, hydroxyzine, promethazine, or a combination thereof; or
guaifenesin, codeine phosphate, dextromethorphan hydrobromide, or a combination thereof; or
dextromethorphan; or
acamprosate, baclofen, buprenorphine, naloxone, clonidine, disulfiram, methadone, naltrexone, ondansetron, or a combination thereof; or
nicotine, bupropion, cytosine, varenicline, or a combination thereof.

12. The gummy composition of Claim 9, wherein the active agent is selected from the following list: amphetamine, dexamfetamine, lisdexamfetamine, methylphenidate, dexmethylphenidate, metamfetamine, or a combination thereof.

13. The gummy composition of Claim 9, wherein the active agent is selected from the following list: citalopram, clomipramine, dexopin, escitalopram, fluoxetine, fluvoxamine, imipramine, mirtazapine, paroxetine, sertraline, trazodone, amitriptyline, doxepin, mianserin, trimipramine, or a combination thereof.

14. The gummy composition of Claim 9, wherein the active agent is selected from the following list: an aldosterone receptor antagonist, an angiotensin converting enzyme inhibitor, an angiotensin receptor blocker, a neprilysin inhibitor, an antiadrenergic agent, an antianginal agent, an antiarrhythmic agent, an anticholinergic chronotropic agent, an antihypertension agent, an ACE inhibitor, an angiotensin II inhibitor, an antiadrenergic agent, a beta blocker, a diuretic agent, a beta-adrenergic blocker, a calcium channel blocker, a catecholamine, an inotropic agent, a vasodilator, a renin inhibitor, a sclerosing agent, a vasopressin antagonist, a vasopressor, an anti-cholesterol agent, or a combination thereof.

15. A medication kit, comprising a gummy composition of Claim 9 and an instruction for using such gummy composition for treating a disease.

## Patentansprüche

1. Verfahren zur Herstellung einer Gummizusammensetzung, das die Bereitstellung einer Geliermischung umfasst, die umfasst:
Pektin mit einem Carboxylgehalt von nicht mehr als 45 Gew.-% des Pektins und einem Amidgehalt von nicht weniger als 20 Gew.-% des Pektins;
eine solubilisierende Mischung, wobei die solubilisierende Mischung ein solubilisierendes Salz mit der Formel M-A umfasst, wobei M ein Kation eines Alkalioder Erdalkalimetalls darstellt und A ein Anion einer mehrwertigen Säure mit mindestens einem pKa-Wert von über 4 darstellt;
ein quellendes Lösungsmittel; und
ein erstes Bindemittelgemisch,
optional die Bereitstellung einer emulgierenden Mischung;
Bereitstellen eines zweiten Bindemittelgemischs, so dass das erste Bindemittelgemisch und das zweite Bindemittelgemisch ein Bindemittelgemisch bilden, wobei das Bindemittelgemisch eine Kombination von mindestens zwei von einem Monosaccharid, einem Disaccharid und einem Trisaccharid umfasst;
Bereitstellen einer aktiven Mischung, die ein aktives Mittel umfasst;
Kombinieren der Geliermischung, der Emulgiermischung, der Bindemischung und der aktiven Mischung, um eine Vorformmischung bereitzustellen;
Kombinieren der Vorformmischung mit einer pHeinstellenden Mischung, um eine Formmischung bereitzustellen, wobei die Formmischung einen pH-Wert von mindestens 5 aufweist; und
Formen der Formmischung zu einer Gummidosierungszusammensetzung.

2. Verfahren nach Anspruch 1, wobei die Geliermischung Pektin mit einem Carboxylgehalt von weniger als 40 Gew.-% des Pektins umfasst.

3. Verfahren nach Anspruch 1, wobei die Geliermischung Pektin mit einem Amidgehalt von 30 bis 35 Gew.-%, bezogen auf Pektin, umfasst.

4. Verfahren nach Anspruch 1, wobei die Geliermischung Pektin mit einem Carboxylgehalt und einem Amidgehalt umfasst, wobei die Summe des Carboxyl- und des Amidgehalts nicht 56 bis 70 Gew.-% des Pektins ausmacht.

5. Verfahren nach Anspruch 1, wobei die Geliermischung Pektin mit einem Carboxylgehalt von nicht mehr als 39 % und einem Amidgehalt von nicht weniger als 27 %, bezogen auf das Gewicht des Pektins, umfasst.

6. Verfahren nach Anspruch 1, wobei das Quellungslösungsmittel Wasser oder eine Kombination aus Wasser und Glycerin umfasst.

7. Verfahren nach Anspruch 1, wobei die Bindemittelmischung eine Kombination von mindestens zwei Monosacchariden, Disacchariden und Trisacchariden umfasst und wobei die Bindemittelmischung Tagatose, Psicose, Trehalose, Isomaltulose oder eine Kombination davon umfasst.

8. Verfahren nach Anspruch 1, wobei das solubilisierende Salz und das Pektin in einem solchen stöchiometrischen Gewichtsverhältnis vorliegen, dass 1/3 der Carboxylgruppen im Pektin dafür konfiguriert ist, deprotoniert zu werden.

9. Gummizusammensetzung, die eine gelierende Mischung, optional eine emulgierende Mischung, eine bindende Mischung und einen Wirkstoff umfasst, wobei die Geliermischung Pektin mit einem Carboxylgehalt von nicht mehr als 45 Gew.-% des Pektins und einem Amidgehalt von nicht weniger als 20 Gew.-% des Pektins umfasst, wobei die Geliermischung ferner eine solubilisierende Mischung umfasst, wobei die solubilisierende Mischung ein solubilisierendes Salz mit einer Formel M-A umfasst, wobei M ein Kation eines Alkali- oder Erdalkalimetalls darstellt und A ein Anion einer mehrwertigen Säure mit mindestens einem pKa größer als 4 darstellt, und wobei die Bindungsmischung eine Kombination von mindestens zwei von einem Monosaccharid, einem Disaccharid und einem Trisaccharid umfasst.

10. Gummizusammensetzung nach Anspruch 9, wobei die Geliermischung ferner Gelatine umfasst, wobei das Kollagen ein MW von 10.000 bis 50.000 Dalton aufweist.

11. Gummizusammensetzung nach Anspruch 9, wobei der Wirkstoff aus der folgenden Liste ausgewählt ist:
ein Penicillin, ein Cephalosporin, ein Makrolid, ein Flurochinolon, ein Sulfonamid, ein Tetracyclin, ein Aminoglycosid oder eine Kombination davon; oder
Acetaminophen, Aspirin, Salicylsäure, Ibuprofen, Naproxen, Diphenhydramin, Scopolamin, Metaformin, ein PDE5-Hemmer, Cetirizin oder eine Kombination davon; oder
Cetirizin, Diphenhydramin, Loratadin, Chlorpheniramin, Brompheniramin, Alimemazin, Cyprohetadin, Doxylamin, Hydroxyzin, Promethazin oder eine Kombination davon; oder
Guaifenesin, Codeinphosphat, Dextromethorphanhydrobromid oder eine Kombination davon; oder
Dextromethorphan; oder
Acamprosat, Baclofen, Buprenorphin, Naloxon, Clonidin, Disulfiram, Methadon, Naltrexon, Ondansetron oder eine Kombination davon; oder
Nikotin, Bupropion, Cytosin, Vareniclin oder eine Kombination davon.

12. Gummizusammensetzung nach Anspruch 9, wobei der Wirkstoff aus der folgenden Liste ausgewählt ist:
Amphetamin, Dexamfetamin, Lisdexamfetamin, Methylphenidat, Dexmethylphenidat, Metamfetamin oder eine Kombination davon.

13. Gummizusammensetzung nach Anspruch 9, wobei der Wirkstoff aus der folgenden Liste ausgewählt ist:
Citalopram, Clomipramin, Dexopin, Escitalopram, Fluoxetin, Fluvoxamin, Imipramin, Mirtazapin, Paroxetin, Sertralin, Trazodon, Amitriptylin, Doxepin, Mianserin, Trimipramin oder eine Kombination davon.

14. Gummizusammensetzung nach Anspruch 9, wobei der Wirkstoff aus der folgenden Liste ausgewählt ist: ein Aldosteron-Rezeptor-Antagonist, ein Angiotensin-Converting-Enzym-Inhibitor, ein Angiotensin-Rezeptor-Blocker, ein Neprilysin-Inhibitor, ein antiadrenerges Mittel, ein antianginöses Mittel, ein antiarrhythmisches Mittel, ein anticholinerges chronotropes Mittel, ein Mittel gegen Bluthochdruck, ein ACE-Hemmer, ein Angiotensin-II-Hemmer, ein antiadrenerges Mittel, ein Betablocker, ein Diuretikum, ein beta-adrenerger Blocker, ein Kalziumkanalblocker, ein Katecholamin, ein inotropes Mittel, ein Vasodilatator, ein Renin-Inhibitor, ein Sklerosierungsmittel, ein Vasopressin-Antagonist, ein Vasopressor, ein Anticholesterinmittel oder eine Kombination davon.

15. Medikamenten-Kit, der eine Gummizusammensetzung nach Anspruch 9 und eine Anleitung zur Verwendung einer solchen Gummizusammensetzung zur Behandlung einer Krankheit umfasst.

## Revendications

1. Procédé de fabrication d'une composition gommeuse, comprenant la fourniture d'un mélange gélifiant, comprenant
de la pectine ayant une teneur en groupes carboxyle ne dépassant pas 45 % en poids de la pectine et une teneur en groupes amide d'au moins 20 % en poids de la pectine ;
un mélange de solubilisation, le mélange de solubilisation comprenant un sel de solubilisation ayant la formule M-A, dans laquelle M représente un cation d'un métal alcalin ou alcalino-terreux et A représente un anion d'un acide multivalent ayant au moins une valeur pKa supérieure à 4 ;
un solvant de gonflement ; et
un premier mélange de liaison,
optionnellement la fourniture d'un mélange émulsifiant ;
la fourniture d'un deuxième mélange de liaison, de telle sorte que le premier mélange de liaison et le deuxième mélange de liaison forment un mélange de liaison, le mélange de liaison comprenant une combinaison d'au moins deux d'un monosaccharide, d'un disaccharide et d'un trisaccharide ;
la fourniture d'un mélange actif, comprenant un agent actif ;
la combinaison du mélange gélifiant, du mélange émulsifiant, du mélange de liaison et du mélange actif pour produire un mélange de pré-moulage ;
la combinaison du mélange de pré-moulage avec un mélange d'ajustement du pH pour produire un mélange de moulage, le mélange de moulage ayant une valeur de pH d'au moins 5 ; et
le formage du mélange de moulage pour produire une composition galénique gommeuse.

2. Procédé selon la revendication 1, dans lequel le mélange gélifiant comprend de la pectine ayant une teneur en groupes carboxyle inférieure à 40 % en poids de la pectine.

3. Procédé selon la revendication 1, dans lequel le mélange gélifiant comprend de la pectine ayant une teneur en groupes amide de 30 % à 35 % en poids de la pectine.

4. Procédé selon la revendication 1, dans lequel le mélange gélifiant comprend de la pectine ayant une teneur en groupes carboxyle et une teneur en groupes amide, le total de la teneur en groupes carboxyle et en groupes amide n'étant pas de 56 % à 70 % en poids de la pectine.

5. Procédé selon la revendication 1, dans lequel le mélange gélifiant comprend de la pectine ayant une teneur en groupes carboxyle ne dépassant pas 39 % et une teneur en groupes amide d'au moins 27 % en poids de la pectine.

6. Procédé selon la revendication 1, dans lequel le solvant de gonflement comprend de l'eau, ou une combinaison d'eau et de glycérol.

7. Procédé selon la revendication 1, dans lequel le mélange de liaison comprend une combinaison d'au moins deux d'un monosaccharide, d'un disaccharide et d'un trisaccharide, et dans lequel le mélange de liaison comprend du tagatose, du psicose, du tréhalose, de l'isomaltulose, ou une combinaison de ceux-ci.

8. Procédé selon la revendication 1, dans lequel le sel de solubilisation et la pectine sont présents selon un rapport poids/poids stoechiométrique tel que 1/3 des groupes carboxyliques dans la pectine sont configurés pour être déprotonés.

9. Composition gommeuse, comprenant un mélange gélifiant, optionnellement un mélange émulsifiant, un mélange de liaison et un agent actif, dans laquelle le mélange gélifiant comprend de la pectine ayant une teneur en groupes carboxyle ne dépassant pas 45 % en poids de la pectine et une teneur en groupes amide d'au moins 20 % en poids de la pectine, dans laquelle le mélange gélifiant comprend en outre un mélange de solubilisation, dans laquelle le mélange de solubilisation comprend un sel de solubilisation ayant la formule M-A, dans laquelle M représente un cation d'un métal alcalin ou alcalino-terreux et A représente un anion d'un acide multivalent ayant au moins une valeur pKa supérieure à 4, et dans laquelle le mélange de liaison comprend une combinaison d'au moins deux d'un monosaccharide, d'un disaccharide et d'un trisaccharide.

10. Composition gommeuse selon la revendication 9, dans laquelle le mélange gélifiant comprend en outre de la gélatine, dans laquelle le collagène a une masse moléculaire (MW) de 10 000 à 50 000 daltons.

11. Composition gommeuse selon la revendication 9, dans laquelle l'agent actif est sélectionné dans la liste suivante :
une pénicilline, une céphalosporine, un macrolide, une fluroquinolone, un sulfamide, une tétracycline, un aminoglycoside, ou une combinaison de ceux-ci ; ou
l'acétaminophène, l'aspirine, l'acide salicylique, l'ibuprofène, le naproxène, la diphénhydramine, la scopolamine, la métaformine, un inhibiteur de la PDE5, la cétirizine, ou une combinaison de ceux-ci ; ou
la cétirizine, la diphénhydramine, la loratadine, la chlorphéniramine, la bromphéniramine, l'alimémazine, la cyprohétadine, la doxylamine, l'hydroxyzine, la prométhazine, ou une combinaison de celles-ci ; ou
la guaïfénésine, le phosphate de codéine, le bromhydrate de dextrométhorphane, ou une combinaison de ceux-ci ; ou
le dextrométhorphane ; ou
l'acamprosate, le baclofène, la buprénorphine, la naloxone, la clonidine, le disulfirame, la méthadone, la naltrexone, l'ondansétron, ou une combinaison de ceux-ci ; ou
la nicotine, le bupropion, la cytosine, la varénicline, ou une combinaison de ceux-ci.

12. Composition gommeuse selon la revendication 9, dans laquelle l'agent actif est sélectionné dans la liste suivante : amphétamine, dexamfétamine, lisdexamfétamine, méthylphénidate, dexméthylphénidate, métamfétamine, ou une combinaison de ceux-ci.

13. Composition gommeuse selon la revendication 9, dans laquelle l'agent actif est sélectionné dans la liste suivante : citalopram, clomipramine, dexopine, escitalopram, fluoxétine, fluvoxamine, imipramine, mirtazapine, paroxétine, sertraline, trazodone, amitriptyline, doxépine, miansérine, trimipramine, ou une combinaison de ceux-ci.

14. Composition gommeuse selon la revendication 9, dans laquelle l'agent actif est sélectionné dans la liste suivante : un antagoniste des récepteurs de l'aldostérone, un inhibiteur de l'enzyme de conversion de l'angiotensine, un bloqueur des récepteurs de l'angiotensine, un inhibiteur de la néprilysine, un agent antiadrénergique, un agent antiangineux, un agent antiarythmique, un agent chronotrope anticholinergique, un agent antihypertenseur, un inhibiteur de l'ACE, un inhibiteur de l'angiotensine II, un agent antiadrénergique, un bêta-bloquant, un agent diurétique, un bêta-bloquant adrénergique, un inhibiteur des canaux calciques, une catécholamine, un agent inotrope, un vasodilatateur, un inhibiteur de la rénine, un agent sclérosant, un antagoniste de la vasopressine, un vasopresseur, un agent médicament anticholestérolémiant, ou une combinaison de ceux-ci.

15. Trousse médicamenteuse, comprenant une composition gommeuse selon la revendication 9 et un mode d'emploi concernant l'utilisation d'une telle composition gommeuse pour le traitement d'une maladie.
